# EUROPEAN PATENT APPLICATION

(11) **EP 3 020 710 A1**
(43) Date of publication of application: **18.05.2016**
(21) Application number: 14822935.4
(22) Date of filing: 11.07.2014
(51) Int. Cl.: C07D 263/20, A61K 31/421, A61P 3/00, A61P 9/00

(54) **OXAZOLIDINONE DERIVATIVES AS PPAR LIGANDS**

(30) Priority: 11.07.2013 ES 201331058
(71) Applicant: Consejo Superior De Investigaciones Científicas (CSIC), 28006 Madrid (ES); Servicio Andaluz de Salud, 41071 Sevilla (ES); Fundación Pública Andaluza Para La Investigación de Malaga en Biomedicina y Salud (FIMABIS), 29010 Málaga (ES)
(72) Inventor: PÉREZ FERNÁNDEZ, Ruth, 28006 Madrid (ES); FRESNO LÓPEZ, María Nieves, 28006 Madrid (ES); ELGUERO BERTOLINI, José, 28006 Madrid (ES); GOYA LAZA, Pilar, 28006 Madrid (ES); TORRES ZAGUIRRE, Ana Belén, 28006 Madrid (ES); RORÍGUEZ DE FONSECA, Fernando, 29010 Málaga (ES); PAVÓN MORÓN, Francisco Javier, 29010 Málaga (ES); MACÍAS GONZÁLEZ, Manuel, 41071 Sevilla (ES); ROMERO CUEVAS, Miguel, 29010 Málaga (ES)
(74) Representative: Hoffmann Eitle
(86) International application number: PCT/ES2014/070572
(87) International publication number: WO 2015/004306

(57) **Abstract**

The present invention relates to a family of differently substituted oxazolidinones and to the pharmaceutically acceptable salts, esters, prodrugs, tautomers, solvates and hydrates thereof, which show affinity for the alpha and gamma subtypes of the peroxisome proliferator-activated receptors (PPAR) and which, therefore, modulate the actions regulated by said receptors, such as inducing satiety, controlling ingestion and modulating metabolic effects, and thus are useful for the administration thereof as a pharmacological tool and as drugs for the treatment and/or prevention of metabolic diseases and cardiovascular diseases.

## Description

### FIELD OF THE INVENTION

The present invention is comprised in the field of pharmaceutical chemistry and pharmacology, and it specifically relates to a family of differently substituted oxazolidinones and to the pharmaceutically acceptable salts, esters, prodrugs, tautomers, solvates, and hydrates thereof, which show affinity for the alpha and gamma subtypes of the peroxisome proliferator-activated receptors (PPAR) and which, therefore, modulate the actions regulated by these receptors, such as inducing satiety, controlling ingestion and modulating metabolic effects, and thus are useful for the administration thereof as a pharmacological tool and as cosmetics and/or drugs for the treatment and/or prevention of metabolic disease and cardiovascular diseases.

### PRIOR ART

PPAR receptors belong to the family of nuclear receptors consisting of three isoforms, alpha, beta and gamma, which are involved in the transcription of genes responsible for regulating metabolism and the energy homeostasis. Their role in regulating the carbohydrate and lipid metabolism is therefore well-established; hence they are useful in the treatment of metabolic disease (S. Alzhar, Future Cardiol. 2010, 6, 657-691).

Activation of the PPAR-alpha (α) subtype by its natural ligands is related to the control of circulating lipid concentrations. Therefore, the alpha subtype of this family of receptors is presented as a very interesting therapeutic target for the treatment of diseases related to metabolic disorders, such as dyslipidemias, and cardiovascular diseases, such as atherosclerosis and hypertension, and obesity (Evans et al., 2004. Nature Medicine 10:355).

In addition, the PPAR gamma (γ) subtype regulates adipogenesis, lipid metabolism, glucose control and inflammation. PPAR gamma activators improve sensitivity to insulin by means of regulating adipogenesis, reducing free fatty acid levels, and improve insulin resistance. However, the selective PPAR gamma agonists have certain side effects, such as peripheral edema or weight gain (A. Tenenbaum, 2012. Cardiovasc Diabetol 11, 140).

Suggestively, when pan activation or dual activation of different PPAR subtypes is performed, different physiological responses occur in a coordinated manner, so pan activation of PPAR gamma and the combined action of alpha can simultaneously lead to insulin resistance and dyslipidemia, making it an interesting target for the treatment of related diseases (A. Tenenbaum 2005. Cardiovasc Diabetol 4,14).

There are already drugs sold on the market that activate PPAR alpha and PPAR gamma. Among such drugs, alpha agonists such as fenofibrate or gemfibrozil are used to treat hyperlipidemia, by regulating different transcription factors involved in some of the dyslipidemia processes (Plutzky J. 2000. Curr. Atheroscler Rep 2: 327). Likewise, thiazolidinediones (TZDs), which are specific gamma receptor ligands, work like "insulin sensitizers" and are sold for hyperglycemia in patients with type 2 diabetes mellitus. In addition to the specific isoform actions, pan agonist or dual agonist agents of PPAR, which act on two or more isoforms, are being described, such as, for example, aleglitazar, which is currently undergoing phase III clinical trials for the treatment of the type 2 diabetes.

In this sense, it is important to point out that the United States Food and Drug Administration determined that, before beginning clinical studies with PPAR agonists, a 2-year study should be done in rodents (http://www.fda.gov/downloads/Drugs/GuidanceComplianceRegulatorylnformation/Guidances/u cm071624.pdf). Therefore, it is important to take into account all the necessary experiments before being applied in humans.

However, fibrates, which bind specifically to the PPAR alpha receptor, are second-choice medicinal products. For safety reasons, they must not be prescribed to patients in whom dyslipidemia is diagnosed for the first time as the first choice with the exception of serious hypertriglyceridemias and in patients who cannot take statins.

In addition, it has been reported that certain chemical substances acting as PPAR gamma agonists cause liver damage and must be used with care, assessing their benefits and side effects.

Compounds that are dual PPAR alpha and PPAR gamma agonists can show fewer therapeutic side effects than those that act only on the PPAR alpha receptor, or those that act exclusively on the PPAR gamma receptor. Therefore, the development of safer and more effective dual PPAR alpha and PPAR gamma agonists has an enormous therapeutic value.

Therefore, there is a need to discover new PPAR agonists. More particularly, there is a need to develop PPAR alpha agonists and PPAR gamma agonists, or dual agonists, suitable for use in therapy. There is an additional need to develop PPAR alpha agonists and PPAR gamma agonists, or dual agonists that have a profile of therapeutically acceptable side effects.

### BRIEF DESCRIPTION OF THE INVENTION

The present invention relates to new chemical structures having nanomolar affinity for PPAR alpha and micromolar affinity for the PPAR gamma subtype, and the novelty of which lies especially in the nature of the terminal end, capable of establishing hydrogen bridge type interactions that are important for activation with the hydroxyl group of the Tyr-464 residue of PPAR alpha and the Tyr-473 residue of PPAR gamma, and which corresponds to a oxazolidinone ring.

A first object of the present invention relates to a compound, hereinafter compound of the invention, of general formula (I): 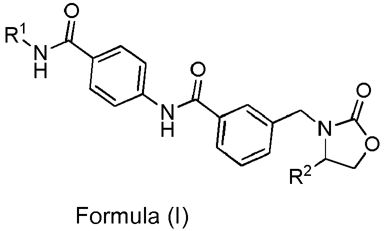 the pharmaceutically acceptable salts, esters, tautomers, prodrugs, solvates and hydrates thereof, where:
R¹ is selected from (C₁-C₁₂) alkyl, cycloalkyl and -(CH₂)-R³, where R³ is cycloalkyl or aryl
R² is selected from (C₁-C₁₂) alkyl, (C₇-C₁₅) aralkyl, aryl and amino
In a preferred embodiment of this object of the invention, R¹ is cycloalkyl.
In another preferred embodiment, R¹ is selected from cyclopropyl, cyclobutyl, cyclopentyl, cyclohexyl and adamantly; in an even more preferred embodiment, R¹ is selected from cyclopropyl and adamantyl.
In another preferred embodiment of the present invention, R¹ is (C₁-C₁₂) alkyl. Even more preferably, R¹ is pentyl or dodecyl.
In another preferred embodiment of the present invention, R¹ is -(CH₂)-R³, where R³ is cycloalkyl or aryl.

In another more preferred embodiment, R³ is cyclohexyl or aryl in the form of 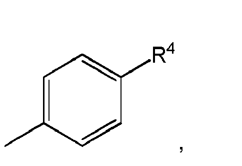 where R⁴ is selected from hydrogen and linear or branched alkyl, which can optionally be substituted with one or several halogens.

In another preferred embodiment of the present invention, R² is (C₇-C₁₅) aralkyl.

In another more preferred embodiment, R² is benzyl.

In other even more preferred embodiments, the compound of formula (I) is selected from the following group:
a) (*R*)-3-((4-benzyl-2-oxooxazolidin-3-yl)methyl)-*N*-(4-((4-(*tert*-butyl)benzyl)carbamoyl)phenyl; benzamide (**1**);
b) (*R*)-3-((4-benzyl-2-oxooxazolidin-3-yl)methyl)-*N*-(4-((4-(trifluoromethyl)benzyl)carbamoyl; phenyl)benzamide (**2**);
c) (*R*)-3-((4-benzyl-2-oxooxazolidin-3-yl)methyl)-*N*-(4-(benzylcarbamoyl)phenyl)benzamide (**3**);
d) (*R*)-3-((4-benzyl-2-oxooxazolidin-3-yl)methyl)-*N*-(4-((cyclohexylmethyl)carbamoyl)phenyl) benzamide (**4**);
e) (R)-3-((4-benzyl-2-oxooxazolidin-3-yl)methyl)-*N*-(4-(pentylcarbamoyl)phenyl)benzamide (**5**);
f) (*R*)-3-((4-benzyl-2-oxooxazolidin-3-yl)methyl)-*N*-(4-(dodecylcarbamoyl)phenyl)benzamide (**6**);
g) (*R*)-3-((4-benzyl-2-oxooxazolidin-3-yl)methyl)-*N*-(4-(cyclopropylcarbamoyl)phenyl)benzamide (**7**); or a pharmaceutically acceptable isomer, ester, pro-drug, tautomer, solvate and/or salt thereof.

In a more preferred embodiment, the compound of the invention is a PPAR α receptor agonist; in another preferred embodiment, the compound of the invention is a PPAR γ receptor agonist; and in an even much more preferred embodiment, the compound of the invention is a dual PPAR α and PPAR γ receptor agonist.

A second object of the present invention relates to a pharmaceutical composition, hereinafter pharmaceutical composition of the invention, comprising a compound of the invention. In a preferred embodiment, the pharmaceutical composition of the invention further comprises at least one pharmaceutically acceptable excipient and a therapeutically effective amount of a compound of formula (I). More preferably, it comprises a pharmaceutically acceptable vehicle, and even more preferably it can further include another active ingredient.

A third object of the present invention relates to a dosage form, hereinafter dosage form of the invention, comprising a compound of the invention, or a composition of the invention.

A fourth object of the present invention relates to the use, preferably *in vitro* use, of a compound of the invention, of a pharmaceutical composition or of a dosage form of the invention as receptor activators of the α (alpha) and γ (gamma) subtypes of the PPAR receptors.

A fifth object of the present invention relates to the use of a compound of the invention, of a pharmaceutical composition or of a dosage form of the invention, in the preparation of a medicinal product.

Therefore, the present invention provides a compound of the invention, a pharmaceutical composition or a dosage form of the invention for use as a medicinal product.

A sixth object of the present invention relates to the use of a compound of the invention, of a pharmaceutical composition or of a dosage form of the invention, in the preparation of a medicinal product for the prevention or treatment of a pathology mediated by the α and γ subtypes of the PPAR receptors (peroxisome proliferator-activated receptors).

Therefore, the present invention provides a compound of the invention, a pharmaceutical composition or a dosage form of the invention for use in a method for the prevention or treatment of a pathology mediated by the α and γ subtypes of the PPAR receptors (peroxisome proliferator-activated receptors).

A seventh object of the present invention relates to the use of a compound of the invention, of a pharmaceutical composition or of a dosage form of the invention, in the preparation of a medicinal product for inducing satiety and controlling ingestion, modulating body fat and regulating lipid and carbohydrate metabolism.

Therefore, the present invention provides a compound of the invention, a pharmaceutical composition or a dosage form of the invention for use in a method for inducing satiety and controlling ingestion, modulating body fat and regulating lipid and carbohydrate metabolism.

An eighth object of the present invention relates to the use of a compound of the invention, of a pharmaceutical composition or of a dosage form of the invention, in the preparation of a medicinal product for the prevention or treatment of a metabolic disease. In a preferred embodiment, the metabolic disease is selected from the group consisting of obesity, dyslipidemia, insulin resistance, hyperglycemia and type 2 diabetes.

Therefore, the present invention provides a compound of the invention, a pharmaceutical composition or a dosage form of the invention for use in a method for the prevention or treatment of a metabolic disease. In a preferred embodiment, the metabolic disease is selected from the group consisting of obesity, dyslipidemia, insulin resistance, hyperglycemia and type 2 diabetes.

A ninth object of the present invention relates to the use of a compound of the invention, of a pharmaceutical composition or of a dosage form of the invention, in the preparation of a medicinal product for the prevention or treatment of a cardiovascular disease. In a preferred embodiment, the cardiovascular disease is selected from atherosclerosis and hypertension.

Therefore, the present invention provides a compound of the invention, a pharmaceutical composition or a dosage form of the invention for use in a method for the prevention or treatment of a cardiovascular disease. In a preferred embodiment, the cardiovascular disease is selected from atherosclerosis and hypertension.

A tenth object of the present invention relates to a cosmetic composition, hereinafter cosmetic composition of the invention, comprising a compound of the invention and at least one cosmetically acceptable excipient and/or adjuvant.

An eleventh object of the present invention relates to a dosage form, hereinafter second dosage form of the invention, comprising the cosmetic composition of the invention.

A twelfth object of the present invention relates to the use of a cosmetic composition of the invention, or of the second dosage form of the invention, for reducing subcutaneous fat.

Therefore, the present invention provides the second dosage form of the invention, a compound of the invention, a pharmaceutical composition or a dosage form of the invention in a method for reducing subcutaneous fat.

A thirteenth object of the present invention relates to a process for preparing the compounds of formula (I) which comprises reacting arylamines (compounds of formula (II)) with esters (compounds of formula (III)), where substituents R¹ and R² are defined as previously indicated.

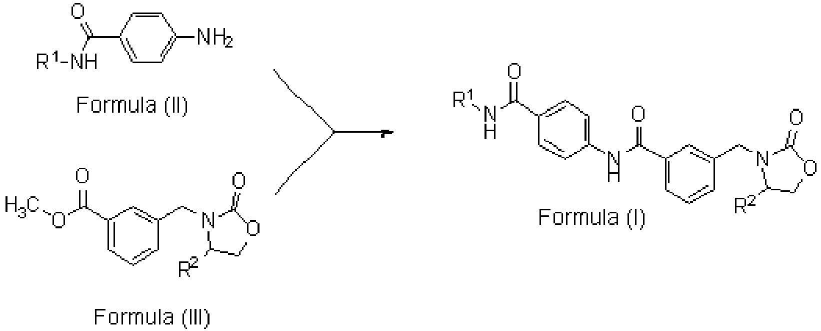

In a preferred embodiment, the method for obtaining a compound of general formula (I) is characterized by the final reaction of an arylamine with an ester, including the following steps:
a) Protecting the amine of p-amino benzoic acid with Boc anhydride;
b) Reacting protected p-amino benzoic acid with an amine by means of HOBt, EDCI or PyBOP acid activating agent in the presence of triethylamine;
c) Deprotecting the amino group in a CH₂Cl₂/TFA biphasic system to obtain an arylamine;
d) Reacting by means of a nucleophilic substitution of an oxazolidinone enolate on the brominated derivative in the presence of potassium *tert*-butoxide as a base in THF to obtain a methyl ester;
e) Reacting to form the amide bond from methyl ester and arylamine in the presence of trimethyl aluminum at 125°C for 35 min in a microwave reactor. The crude reaction product is cooled in an ice-H₂O mixture and acidified with HCl (1 N), until effervescence stops;
f) Extracting with diethylether (2 x 20 ml), drying over anhydrous MgSO₄ and removing the solvent at reduced pressure;
g) Purifying the crude reaction product by chromatography (6:4 Hex/AcOEt).

More preferably, the method for obtaining a compound of general formula (I) is characterized in that the amine is chosen from 4-(*tert*-butyl)-benzylamine, 4-(trifluoromethyl)-benzylamine, benzylamine, hexahydrobenzylamine, 1-pentylamine, 1-dodecylamine, cyclopropylamine, and the ester is methyl 3-(bromomethyl)benzoate.

The present invention furthermore provides a composition comprising a compound of the invention.

The present invention furthermore provides a nutraceutical composition comprising a compound of the invention.

The present invention furthermore provides the use of the nutraceutical composition of the invention for reducing subcutaneous fat.

### BRIEF DESCRIPTION OF THE DRAWINGS

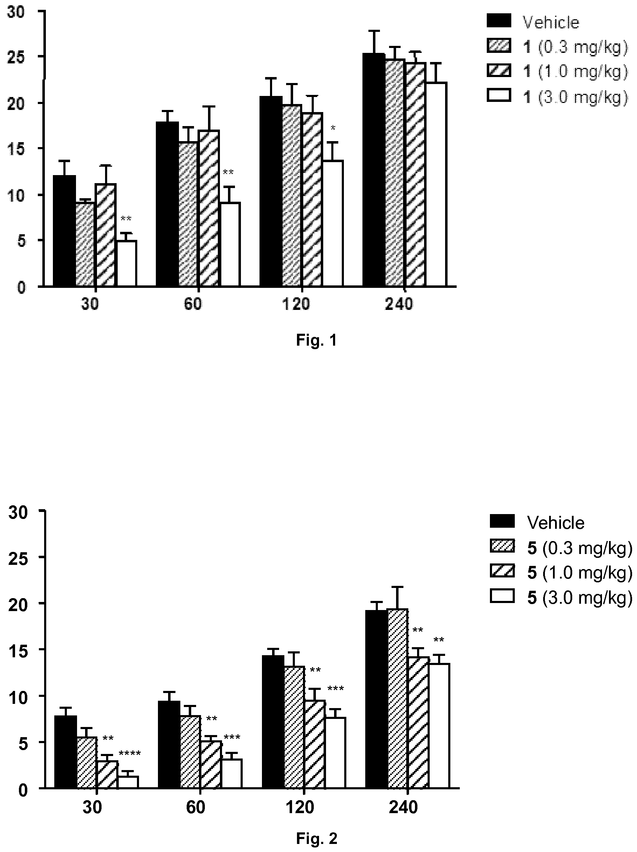

Figure 1 shows the results of the acute ingestion experiment in relation to compound 1. The normalized values per gram of food consumed with respect to kg of animal are represented. The compound is active up to 120 min after the administration thereof. The x-axis represents time (min). The y-axis represents accumulated food ingestion (g/kg).
Figure 2 shows the results of the acute ingestion experiment in relation to compound 5. The normalized values per gram of food consumed with respect to kg of animal are represented. The compound is active in a dose-dependent manner. The x-axis represents time (min). The y-axis represents accumulated food ingestion (g/kg).

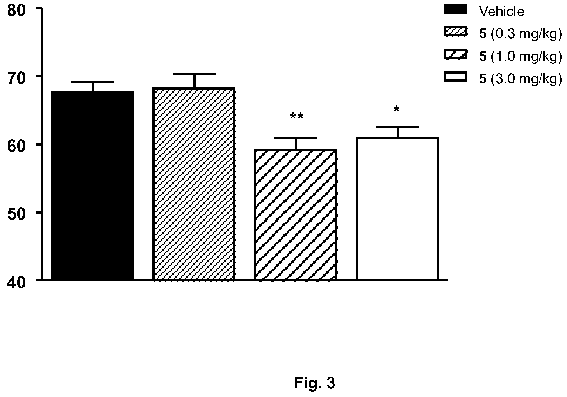

Figure 3 shows the results of ingestion at 24 hours after administration of compound 5. Is represented the normalized values per gram of food consumed with respect to kg of animal. The compound is still active after 24 hours in a dose-dependent manner. The x-axis represents time of treatment (24 hours), and the y-axis represents accumulated food ingestion (g/kg).

### DETAILED DESCRIPTION OF THE INVENTION

The authors of the present invention have found that the compounds of formula (I) surprisingly activate the alpha and gamma subtypes of PPAR receptors in the nanomolar and micromolar ranges, respectively.

In a first aspect, the present invention relates to a compound of general formula (I) 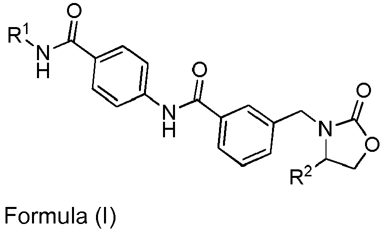 the pharmaceutically acceptable salts, esters, tautomers, prodrugs, solvates and hydrates thereof.

R¹ is selected from (C₁-C₁₂) alkyl, cycloalkyl and -(CH₂)-R³ where R³ is cycloalkyl or aryl.

R² is selected from alkyl, (C₇-C₁₅) aralkyl, aryl and amino.

According to a preferred embodiment of the present invention, R¹ is cycloalkyl.

In another preferred embodiment, R¹ is selected from cyclopropyl, cyclobutyl, cyclopentyl and cyclohexyl.

In an even more preferred embodiment, R¹ is cyclopropyl or adamantyl.

According to another preferred embodiment of the present invention, R¹ is (C₁-C₁₂) alkyl.

In another more preferred embodiment, R¹ is pentyl or dodecyl.

According to another preferred embodiment of the present invention, R¹ is -(CH₂)-R³, where R³ is cycloalkyl or aryl.

In another more preferred embodiment, R³ is cyclohexyl or aryl in the form of 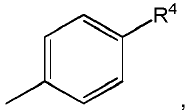 where R⁴ is selected from hydrogen and linear or branched alkyl, which can optionally be substituted with one or several halogens.

According to another preferred embodiment of the present invention, R² is (C₇-C₁₅) aralkyl. In another more preferred embodiment, R² is benzyl.

According to a more preferred embodiment, the compound of formula (I) is selected from the following group:
a) (*R*)-3-((4-benzyl-2-oxooxazolidin-3-yl)methyl)-*N*-(4-((4-(*tert-*butyl)benzyl)carbamoyl)phenyl)benzamide (**1**) (Formula IV);
b) (*R*)-3-((4-benzyl-2-oxooxazolidin-3-yl)methyl)-*N*-(4-((4-(trifluoromethyl)benzyl)carbamoyl)phenyl)benzamide (**2**) (Formula V);
c) (*R*)-3-((4-benzyl-2-oxooxazolidin-3-yl)methyl)-*N*-(4-(benzylcarbamoyl)phenyl)benzamide (**3)** (Formula VI);
d) (*R*)-3-((4-benzyl-2-oxooxazolidin-3-yl)methyl)-*N*-(4-((cyclohexylmethyl)carbamoyl)phenyl)benzamide (**4**) (Formula VII);
e) (R)-3-((4-benzyl-2-oxooxazolidin-3-yl)methyl)-*N*-(4-(pentylcarbamoyl)phenyl)benzamide (**5**) (Formula VIII);
f) (*R*)-3-((4-benzyl-2-oxooxazolidin-3-yl)methyl)-*N*-(4-(dodecylcarbamoyl)phenyl)benzamide (**6**) (Formula IX);
g) (*R*)-3-((4-benzyl-2-oxooxazolidin-3-yl)methyl)-*N*-(4-(cyclopropylcarbamoyl)phenyl)benzamide (**7**) (Formula X);
or a pharmaceutically acceptable isomer, prodrug, tautomer, solvate and/or salt thereof.

The term "(C₁-C₁₂) alkyl" refers, in the present invention, to radicals of linear or branched hydrocarbon chains, consisting of carbon and hydrogen atoms, which do not have unsaturation, having from one to twelve carbon atoms and which are bound to the rest of the molecule by a single bond, for example, methyl, ethyl, n-propyl, *i*-propyl, *n*-butyl, *t*-butyl, *n*-pentyl, etc. The alkyl radicals can be optionally substituted with one or more substituents, such as an aryl, cycloalkyl, halogen, hydroxyl, alkoxyl, carboxyl, cyano, carbonyl, acyl, alkoxycarbonyl, amino, nitro, mercapto, alkylthio, etc.

The term (C₇-C₁₅) aralkyl" refers, in the present invention, to an alkyl radical substituted with an aryl radical, the aryl radical being able to have other substituents, such as a halogen, for example. Examples of aralkyls are benzyl and phenethyl.

The term "cycloalkyl" refers to a stable monocyclic, bicyclic or tricyclic radical having from 3 to 10 members, which is saturated or partially saturated, and which only consists of carbon and hydrogen atoms, such as cyclohexyl or adamantyl. Unless indicated otherwise in the specification, the term "cycloalkyl" refers to including radicals cycloalkyl which are optionally substituted with one or more substituents such as alkyl, halogen, hydroxyl, amino, cyano, nitro, alkoxyl, carboxyl, alkoxycarbonyl, etc.

The term "aryl" refers, in the present invention, to radicals of single or multiple aromatic rings, having from 5 to 18 links in which a proton has been removed from the ring. The aryl groups are, for example, but not being limited to, phenyl, naphthyl. Preferably the aryl group has from 5 to 7 carbon atoms, and more preferably the aryl group is a phenyl. The aryl radicals can be optionally substituted with one or more substituents linear or branched such as (C₁-C₆) alkyl and optionally substituted with a halogen, trifluoroalkyl, halogen, hydroxyl or carboxylic acid.

The term "halogen" refers, in the present invention, to bromine, chlorine, iodine or fluorine. In a preferred embodiment, it refers to fluorine.

Unless indicated otherwise, the compounds of the invention also refer to compounds differing only by the presence of one or more isotopically enriched atoms. For example, the compounds having the present structures, with the exception of the substitution of a hydrogen with a deuterium or with tritium, or the substitution of a carbon with a ¹³C- or ¹⁴C-enriched carbon, or a ¹⁵N-enriched nitrogen, are within the scope of this invention.

The term "tautomer" or "tautomeric form", as it is used in the present invention, refers to structural isomers of different energies which are interconvertible via a low-energy barrier. For example, proton tautomers (also known as prototropic tautomers) including interconversions by means of the migration of a proton, such as, for example, keto-enol or imine-enamine isomerizations. Valence tautomers include interconversions by reorganizing a number of bonding electrons.

The term "pharmaceutically acceptable salt and/or solvate" refers to any pharmaceutically acceptable salt, ester, solvate, or any other pharmaceutically acceptable compound which, when administered to a recipient is capable of providing (directly or indirectly) a compound as described herein. However, it will be seen that pharmaceutically unacceptable salts also are within the scope of the invention because they can be useful in the preparation of pharmaceutically acceptable salts. The term "pharmaceutically acceptable" refers to molecular entities and compositions which are tolerable physiologically and typically do not cause an allergic reaction or similar unfavorable reaction, such as a stomach disorder, dizziness and the like, when administered to a human. Preferably, the term "pharmaceutically acceptable" means tested by a regulatory agency of a federal or state government or included in the US Pharmacopoeia or other generally well-known pharmacopoeia for use in animals, and more particularly in humans. The preparation of salts and solvates can be carried out by means of methods known in the art.

For example, pharmaceutically acceptable salts of compounds provided herein are synthesized by means of conventional chemical methods from an original compound containing a base or acid residue. Generally, such salts are prepared, for example, by reacting the free base or acid forms of the compounds with a stoichiometric amount of the suitable base or acid in water or in an organic solvent or in a mixture of both. Generally, non-aqueous media such as ether, ethyl acetate, ethanol, isopropanol or acetonitrile are preferred. Examples of acid addition salts include mineral acid addition salts such as, for example, hydrochloride, hydrobromide, hydroiodide, sulfate, nitrate, phosphate, and organic acid addition salts such as, for example, acetate, maleate, fumarate, citrate, oxalate, succinate, tartrate, malate, mandelate, methanesulfonate and p-toluenesulfonate. Examples of base addition salts include inorganic salts such as, for example, of sodium, potassium, calcium, ammonium, magnesium, aluminum and lithium salts, and organic base salts such as, for example, ethylenediamine, ethanolamine, *N*,*N*-dialkylenethanolamine, triethanolamine, glucamine, and basic amino acid salts.

Any compound which is a prodrug of a compound of formula (I) is within the scope of the invention. The term "prodrug" is used in its broadest sense and covers those derivatives that are converted into the compounds of the invention *in vivo.* Such derivatives will be evident for those skilled in the art and include, depending on the functional groups present in the molecule and without limitation, the following derivatives of the compounds have esters, amino acid esters, phosphate esters, sulfonate esters of metal salts, carbamates and amides.

The particularly preferred prodrug derivatives are those which increase the bioavailability of the compounds of this invention when such compounds are administered to a patient (for example, making an orally administered compound be more readily absorbed by the blood), or which enhance the release of the original compound in a biological compartment (for example, the brain or lymphatic system) in relation to the original species.

The compounds of formula (I) can be in crystalline form as free compounds or as solvates, and the intention is for both forms to be within the scope of the present invention. Solvation methods are known generally in the art. Suitable solvates are pharmaceutically acceptable solvates. In a particular embodiment, the solvate is a hydrate.

The compounds of formula (I) or the salts or solvates thereof are preferably in a pharmaceutically acceptable or substantially pure form. Pharmaceutically acceptable form is understood, among others, as having a level of pharmaceutically acceptable purity, excluding the normal pharmaceutical additives such as diluents and carriers, and not including material considered to be toxic at normal dosage levels. Purity levels for the active ingredient are preferably greater than 50%, more preferably greater than 70%, more preferably greater than 90%. In a preferred embodiment, they are greater than 95% of the compound of formula (I), or of the salts, solvates or prodrugs thereof.

The compounds of formula (I) can contain one or more asymmetrical centers or stereocenters and can be presented as racemic mixtures, optically enriched mixtures, pure enantiomers or diastereomeric mixtures. The present invention includes all the isomers of the compounds of formula (I) and mixtures of compounds. When the structures are shown with a given stereochemistry, the other stereochemistries are included as the enantiomers or diastereoisomers thereof, as well as the mixtures of enantiomers and/or diastereomers, including racemic mixtures. The compounds of formula (I) having one or more stereocenters can be separated into their corresponding enantiomers and diastereomers by methods known in the prior art. Alternatively, enantiomers and other compounds with stereogenic centers can be synthesized from optically pure starting materials having a known configuration.

It has been found in the present invention that the compounds of the invention show modulatory activity on PPAR receptors. Therefore, the compounds of formula (I) and the therapeutically acceptable salts, prodrugs, tautomers or solvates thereof can be used in the prevention and/or treatment of a disorder or disease in which modulation of said receptors is relevant.

A second object of the present invention relates to a pharmaceutical composition, hereinafter pharmaceutical composition of the invention, comprising a compound of the invention. In a preferred embodiment, the pharmaceutical composition of the invention further comprises at least one pharmaceutically acceptable excipient and a therapeutically effective amount of a compound of formula (I). More preferably, it comprises a pharmaceutically acceptable vehicle, and even more preferably it can further include another active ingredient.

The compositions of the present invention can be formulated for the administration thereof to an animal, and more preferably to a mammal, including humans, in a range of forms known in the prior art. It can therefor be, without limitation, in a sterile aqueous solution or in biological fluids, such as serum. The aqueous solutions can be buffered or non-buffered and have additional active or inactive components. The additional components include salts for modulating ionic strength, preservatives including, but not being limited to, agents antimicrobial, antioxidants, chelating agents, and the like, and nutrients including glucose, dextrose, vitamins and minerals. Alternatively, the compositions can be prepared for the administration thereof in solid form. The compositions can be combined with several inert vehicles or excipients, including, but not being limited to, binders such as microcrystalline cellulose, tragacanth gum, or gelatin; excipients such as starch or lactose; dispersing agents such as alginic acid or corn starch; lubricants such as magnesium stearate, glidants such as colloidal silicon dioxide; sweetening agents such as sucrose or saccharine; or flavoring agents such as mint or methyl salicylate.

Therefore, in a preferred embodiment of this aspect of the invention, the pharmaceutical composition can further comprise a pharmaceutically acceptable vehicle. In another preferred embodiment, the pharmaceutical composition further comprises excipients. In another preferred embodiment the pharmaceutical composition can further comprise another active ingredient.

As used herein, the term "active ingredient", "active substance", "pharmaceutically active substance", or "pharmaceutically active ingredient" means any component which potentially provides a pharmacological activity or another different effect in the diagnosis, cure, mitigation, treatment, or prevention of a disease, or which affects the structure or function of the body of humans or other animals. The term includes those components promoting a chemical change in the preparation of the drug and are present therein in a foreseen modified manner providing the specific activity or effect.

A third object of the present invention relates to a dosage form, hereinafter dosage form of the invention, comprising a compound of the invention, or a composition of the invention.

In this specification, "dosage form" is understood as the mixture of one or more active ingredients with or without additives having physical characteristics for the suitable dosage, conservation, administration and bioavailability thereof.

The term "therapeutically effective amount" means the amount of a compound required for the treatment or prevention of the disease, disorder or condition to be effective.

The compounds, pharmaceutical compositions and dosage forms of this invention can be used alone or together with other drugs to provide a combined therapy. The other drugs can be part of the same pharmaceutical composition or be provided as a separate pharmaceutical composition for the administration thereof at the same time or at a different time. Examples of pharmaceutical compositions include any solid composition (tablets, pills, capsules, granules, etc.) or liquid composition (solutions, suspensions or emulsions) for oral, topical or parenteral administration. The compositions and/or their formulations can be administered to an animal, including a mammal, and therefore to humans, in a range of forms, including, but not being limited to, intraperitoneal, intravenous, intramuscular, subcutaneous, intrathecal, intraventricular, oral, enteral, parenteral, intranasal or topical form. In a preferred embodiment, the pharmaceutical composition is suitable for oral administration.

A fourth object of the present invention relates to the use, preferably *in vitro* use, of a compound of the invention, of a pharmaceutical composition or of a dosage form of the invention as receptor activators of the α (alpha) and γ (gamma) subtypes of the PPAR receptors.

A fifth object of the present invention relates to the use of a compound of the invention, of a pharmaceutical composition or of a dosage form of the invention, in the preparation of a medicinal product, or alternatively, a compound of the invention, of a pharmaceutical composition or of a dosage form of the invention for use as a medicinal product.

The term "medicinal product", as it is used in this specification, refers to any substance used for prevention, diagnosis, alleviation, treatment or cure of diseases in humans and animals. In the context of the present invention, the disease is a pathology mediated by the α and γ subtypes of the PPAR receptors (peroxisome proliferator-activated receptors).

Therefore, a sixth object of the present invention relates to the use of a compound of the invention, of a pharmaceutical composition or of a dosage form of the invention, in the preparation of a medicinal product for the prevention or treatment of a pathology mediated by the α and γ subtypes of the PPAR receptors (peroxisome proliferator-activated receptors).

Therefore, the present invention provides a compound of the invention, a pharmaceutical composition or a dosage form of the invention for use in a method for the prevention or treatment of a pathology mediated by the α and γ subtypes of the PPAR receptors (peroxisome proliferator-activated receptors).

A seventh object of the present invention relates to the use of a compound of the invention, of a pharmaceutical composition or of a dosage form of the invention, in the preparation of a medicinal product for inducing satiety and controlling ingestion, modulating body fat and regulating lipid and carbohydrate metabolism.

Therefore, the present invention provides a compound of the invention, a pharmaceutical composition or a dosage form of the invention for use in a method for inducing satiety and controlling ingestion, modulating body fat and regulating lipid and carbohydrate metabolism.

An eighth object of the present invention relates to the use of a compound of the invention, of a pharmaceutical composition or of a dosage form of the invention, in the preparation of a medicinal product for the prevention or treatment of a metabolic disease. In a preferred embodiment, the metabolic disease is selected from the group consisting of obesity, dyslipidemia, insulin resistance, hyperglycemia and type 2 diabetes.

Therefore, the present invention provides a compound of the invention, a pharmaceutical composition or a dosage form of the invention for use in a method for the prevention or treatment of a metabolic disease. In a preferred embodiment, the metabolic disease is selected from the group consisting of obesity, dyslipidemia, insulin resistance, hyperglycemia and type 2 diabetes.

A ninth object of the present invention relates to the use of a compound of the invention, of a pharmaceutical composition or of a dosage form of the invention, in the preparation of a medicinal product for the prevention or treatment of a cardiovascular disease. In a preferred embodiment, the cardiovascular disease is selected from atherosclerosis and hypertension.

Therefore, the present invention provides a compound of the invention, a pharmaceutical composition or a dosage form of the invention for use in a method for the prevention or treatment of a cardiovascular disease. In a preferred embodiment, the cardiovascular disease is selected from atherosclerosis and hypertension.

A tenth object of the present invention relates to a cosmetic composition, hereinafter cosmetic composition of the invention, comprising a compound of the invention and at least one cosmetically acceptable excipient and/or adjuvant.

The therapeutically effective amount of compound of formula (I), the pharmaceutically acceptable salts, prodrugs or solvates thereof, which must be administered as well as the dosage thereof to treat a pathological condition with said compounds will depend on a number of factors, including the patient's age and condition, the severity of the disease, the administration route and frequency, the modulating compound to be used, etc.

The compounds and compositions of this invention can be used alone or together with other drugs to provide a combined therapy. The other drugs can be part of the same composition or be provided as a separate composition, for the administration thereof at the same time or at a different time.

An eleventh object of the present invention relates to a dosage form, hereinafter second dosage form of the invention, comprising the cosmetic composition of the invention. Said composition can be presented in any suitable application form, for example, solid, liquid or semisolid, such as a cream, ointment, gel or solution, and can be applied by any suitable route, preferably topically, for which purpose it would include the cosmetically acceptable adjuvants required for the formulation of the desired form of administration.

A twelfth object of the present invention relates to the use of a cosmetic composition of the invention, or of the second dosage form of the invention, for reducing subcutaneous fat.

Therefore, the present invention provides the second dosage form of the invention, a compound of the invention, a pharmaceutical composition or a dosage form of the invention in a method for reducing subcutaneous fat.

A thirteenth object of the present invention relates to a process for preparing the compounds of formula (I) which comprises reacting arylamines (compounds of formula (II)) with esters (compounds of formula (III)) where the substituents R¹ and R² are defined as previously indicated.

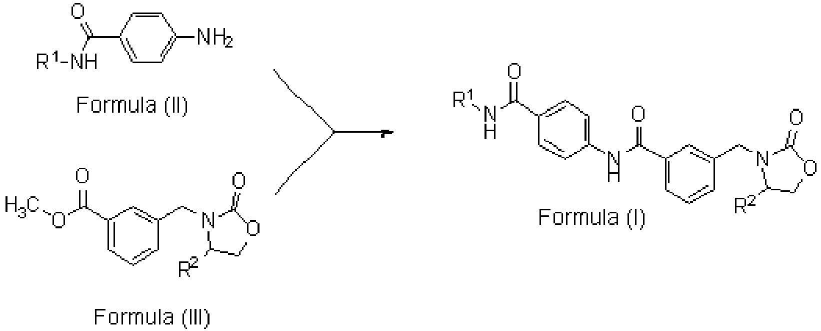

In a preferred embodiment, the method for obtaining a compound of general formula (I) is characterized by the final reaction of an arylamine with an ester, including the following steps:
a) Protecting the amine of p-amino benzoic acid with Boc anhydride;
b) Reacting protected p-amino benzoic acid with an amine by means of HOBt, EDCI or PyBOP acid activating agent in the presence of triethylamine;
c) Deprotecting the amino group in a CH₂Cl₂/TFA biphasic system to obtain an arylamine;
d) Reacting by means of a nucleophilic substitution of an oxazolidinone enolate on the brominated derivative in the presence of potassium *tert*-butoxide as a base in THF to obtain a methyl ester;
e) Reacting to form the amide bond from methyl ester and arylamine in the presence of trimethyl aluminum at 125°C for 35 min in a microwave reactor. The crude reaction product is cooled in an ice-H₂O mixture and acidified with HCl (1 N), until effervescence stops;
f) Extracting with diethylether (2 x 20 ml), drying over anhydrous MgSO₄ and removing the solvent at reduced pressure;
g) Purifying the crude reaction product by chromatography (6:4 Hex/AcOEt).

More preferably, the method for obtaining a compound of general formula (I) is characterized in that the amine is chosen from 4-(*tert*-butyl)-benzylamine, 4-(trifluoromethyl)-benzylamine, benzylamine, hexahydrobenzylamine, 1-pentylamine, 1-dodecylamine, cyclopropylamine and the ester is methyl 3-(bromomethyl)benzoate.
The present invention furthermore provides a composition comprising a compound of the invention.

The present invention furthermore provides a nutraceutical composition comprising a compound of the invention.

A nutraceutical composition, as it is used in this specification, refers to any composition or ingredient thereof providing a benefit for human health. They are preferably food compositions and/or functional foods, i.e., foods which are prepared not only due to their characteristics nutritional but also because they comply with a specific function, such as improving health and reducing the risk of contracting diseases.

Preferred food compositions are selected from, but not limited to, beverages, foods, infusions, milk, yogurt, cheese, fermented milk, milk-flavored beverages, soy milk, cereals, bread, cake, butter, margarine, sauces, frying oils, vegetable oils, corn oil, olive oil, soybean oil, palm oil, sunflower oil, cottonseed oil, condiments, salad dressings, fruit juices, syrups, desserts, glazes and fillings, soft frozen products, sweets, and chewing gum.

The present invention furthermore provides the use of the nutraceutical composition of the invention for reducing subcutaneous fat.

### EXAMPLES

The following examples are provided by way of illustration and do not intend to limit the present invention.

### A. CHEMICAL SYNTHESIS

For the synthesis of the compounds of formula (I) of the present invention has been followed a convergent synthesis route in which the final compounds are the result of the binding of the compounds of formula (II) to the compounds of formula (III).

This method is summarized in the following scheme (I): 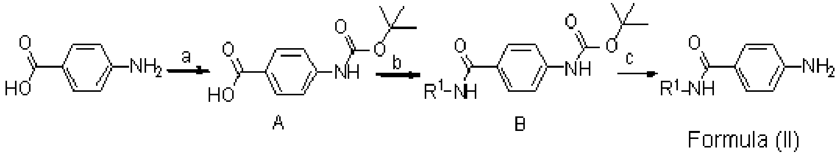 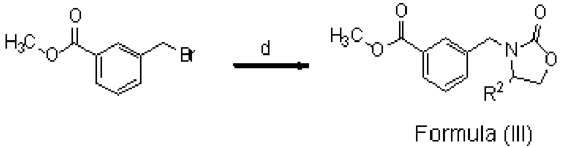 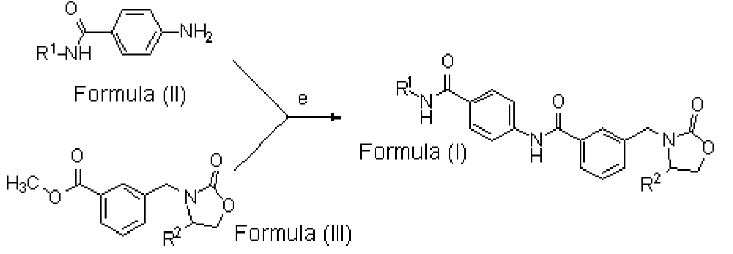 a) Boc₂O, Et₃N, H₂O/dioxane; b) PyBOP, HOBt, Et₃N, THF; c) CH₂Cl₂/TFA; d) (*R*)-(-)-4-R²-3-propionyloxazolidin-2-one, ^{t}BuOK, THF; e) Al(CH₃)₃ in 2.0 M heptane, THF.

### SCHEME (I)

For the synthesis of the compounds of formula (II), p-amino benzoic acid in which the amine thereof is protected with Boc anhydride has been used as the starting material. The resulting intermediate (A) reacts with differently substituted amines (R¹-NH₂) by means of HOBt, EDCI or PyBOP acid activating agent in the presence of triethylamine giving rise to intermediate (B). The amino group is deprotected in a CH₂Cl₂/TFA biphasic system providing the compounds of formula (II). Then, the synthesis of the compounds of formula (III) is performed by means of a nucleophilic substitution of the enolate of the corresponding oxazolidinone on the brominated derivative in the presence of potassium *tert*-butoxide as a base in THF.

The binding of the compounds of formula (II) and (III) by means of the formation of the amide bond from the methyl ester in the presence of trimethyl aluminum gives rise to the family of compounds of general formula (I).

### Example 1. Preparing and obtaining (R)-3-((4-benzyl-2-oxooxazolidin-3-yl)methyl)-N-(4-((4-(tert-butyl)benzyl)carbamoyl)phenyl)benzamide (1), of formula (IV)

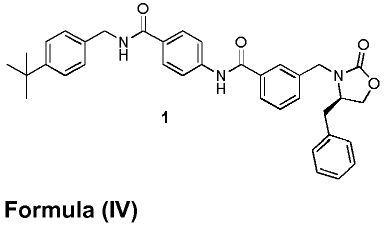

Al(CH₃)₃ in 2.0 M heptane (0.18 ml, 0.36 mmol), under N₂ atmosphere, is added to a solution of (*R*)-3-((4-benzyl-2-oxooxazolidin-3-yl)methyl)methyl benzoate (0.06 g, 0.18 mmol) and the compound 4-amino-*N*-(4-(*tert*-butyl)benzyl)benzamide (0.10 g, 0.36 mmol); the reaction mixture is heated at 125°C for 35 min in a microwave reactor. The crude reaction product is cooled in an ice-H₂O mixture and acidified with HCl (1 N) until effervescence stops. Then, it is extracted with diethylether (20 ml), the organic extracts are dried over anhydrous MgSO₄, and the solvent is removed at reduced pressure. The crude reaction product is purified by biotage chromatography (Hex/AcOEt), obtaining **1** as a white solid (0.05 g, 44%). ¹H NMR (300 MHz, CDCl₃) δ 8.91 (s, 1 H, NH), 7.83 (m, 1 H), 7.78 - 7.63 (m, 5H), 7.44 - 7.15 (m, 9H), 7.07 - 6.96 (m, 2H), 6.66 (t, *J* = 5.7 Hz, 1 H, NHCH₂), 4.74 (m, 1 H), 4.57 (d, *J* = 5.7 Hz, 2H, NHCH₂), 4.21 - 4.07 (m, 2H), 4.00 (m, 1H), 3.87 (m, 1 H, H-4), 3.04 (dd, *J* = 13.6,4.8 Hz, 1H, H-6), 2.63 (dd, *J=* 13.6, 8.5 Hz, 1 H, H-6), 1.30 (s, 9H, 3CH₃). ¹³C NMR (75 MHz, CDCl₃) δ 166.8 (CO), 165.8 (CO), 158.6 (C-2), 150.5, 141.2 (C-7), 136.5 (C-14), 135.2, 131.2, 129.8, 129.2, 128.9 (2C), 128.9 (2C), 127.9 (2C), 127.7 (2C), 127.2, 127.1, 126.6, 125.6 (2C), 119.9 (2C), 67.1 (C-5), 55.8 (C-4), 46.0 (C-13), 43.8 (CH₂), 38.6 (C-6), 34.5 (C(CH₃)₃), 31.3 (3CH₃). Theoretical elemental analysis C₃₆H₃₇N₃O_{4:} C, 75.11; H, 6.48; N, 7.30; experimental elemental analysis C, 75.00; H, 6.75; N, 7.70.

### 1) Intermediate (A): 4-[(tert-butoxycarbonyl)amino] benzoic acid

Triethylamine (0.4 ml, 3 mmol) followed by di-t*ert*-butyldicarbonate (0.6 g, 3 mmol) is added to a mixture of 4-aminobenzoic acid (0.2 g, 1.5 mmol) in dioxane (4 ml) and H₂O (2 ml). The reaction mixture is stirred at room temperature for 24 h. The solvent is removed at reduced pressure, and the residue is acidified with 1 N HCl. The precipitate obtained is washed with H₂O, obtaining **I** as a white solid (0.4 g, 89%). ¹H NMR (300 MHz, DMSO-d₆) δ 9.71 (s, 1 H, COOH), 7.82 (d, *J =* 8.7 Hz, H-2,6), 7.54 (d, *J*=9 Hz, H-3,5), 1.44 (s, 9H, 3CH₃). ¹³C NMR (75 MHz, DMSO-*d*₆) δ 167.4 (CO), 152.9 (CO), 144.1, 130.3 (2CHar), 124.4, 117.6 (2CHar), 80.0 (C(CH₃)₃), 28.4 (3CH₃). HPLC gradient of 15-95% CH₃CN/H₂O in 10 min, tr= 4.25 min, m/z [M+H]⁺= 237. Theoretical elemental analysis C₁₂H₁₅NO₄: C, 60.75; H, 6.37; N, 5.90; experimental elemental analysis C, 60.84; H, 6.09; N, 5.95.

### 2) Intermediate (B): 4-[N-(tert-butoxycarbonyl)amino]-N-(tert-butyl)benzylamide

PyBOP (0.79 g, 1.52 mmol), Et₃N (0.21 ml, 1.52 mmol) and HOBt (0.21 g, 1.52 mmol) are added to a solution of **4-[(*tert*-butoxycarbonyl)amino]** benzoic acid (0.30 g, 1.26 mmol) in THF; the reaction mixture is cooled at 0°C for several minutes. Then, 4-(*tert*-butyl)-benzylamine (0.08 g, 1.52 mmol) is added, and it is maintained with constant stirring at room temperature for 12 h. The solvent is removed at reduced pressure, the residue is acidified with 1 N HCl (20 ml) and extracted with DCM (20 ml). The organic extracts are dried over anhydrous MgSO₄, and the solvent is removed at reduced pressure. The crude reaction product is purified by column chromatography (6:4 hexane/ethyl acetate), obtaining **II** as a white solid (0.3 g, 60%). ¹H NMR (300 MHz, CDCl₃) δ 7.72 (d, *J* = 8.6 Hz, 2H, H-2,6), 7.42 (d, *J*= 8.6 Hz, 2H, H-3,5), 7.38 (d, *J =* 8.4 Hz, 2H, H-1 ',3'), 7.29 (d, J= 8.4 Hz, 2H, H-3', 6') 6.77 (s, 1 H, NH), 6.37 (t, *J* = 5.4 Hz, 1 H, NHCH₂), 4.59 (d, *J* = 5.4 Hz, 2H, NHCH₂), 1.51 (s, 9H, CH₃), 1.31 (s, 9H, CH₃). ¹³C NMR (75 MHz, CDCl₃) δ 166.7 (CO), 152.3 (CO), 150.6, 141.5, 135.2, 128.5, 128.0 (C-3,5), 127.8 (C-3',5'), 125.7 (C-2,6), 117.7 (C-2',6'), 81.0 (C(CH₃)₃), 43.8 (CH₂), 34.5 (C), 31.3 (3CH₃), 28.3 (3CH₃). HPLC gradient of 15-95% CH₃CN/H₂O in 5 min, tr= 5.71 min, m/z [M+H]⁺= 383. Theoretical elemental analysis C₂₃H₃₀N₂O₃: C, 72.22; H, 7.91; N, 7.32; experimental elemental analysis C, 72.51; H, 7.95; N, 7.20.

### 3) Formula (II):4-amino-N-(4-(tert-butyl)benzyl)benzamide

20 ml of trifluoroacetic acid are added to a solution of 4-[*N*-(*tert*-butoxycarbonyl)amino]-*N*-(*tert-*butyl)benzylamide (0.20 g, 0.52 mol) in DCM (20 ml); the reaction mixture is maintained with constant stirring and at room temperature for 5 h. Then, the solvent is removed at reduced pressure, and the crude reaction product is dissolved in a 1 M DCM/NaOH biphasic system (1:1) (20 ml); the mixture is stirred for 10 min. Then, it is extracted with DCM, and the organic extracts are dried over MgSO₄. The solvent is removed at reduced pressure obtaining **III** as a white solid (0.13 g, 90%). ¹H NMR (300 MHz, CDCl₃) δ 7.62 (d, *J* = 8.3 Hz, 2H, H-3', 5'), 7.37 (d, *J* = 8.3 Hz, 2H, H-3', 5'), 7.29 (d, *J* = 8.4 Hz, 2H, H-2', 6'), 6.64 (d, *J* = 8.4 Hz, 2H, H-2,6), 4.59 (d, *J =* 5.4 Hz, 2H, NHCH₂), 1.32 (s, 6H, CH₃). ¹³C NMR (75 MHz, CDCl₃) δ 167.0 (CO), 150.5, 149.5, 135.5, 128.7 (C-3,5), 127.7 (C-3',5'), 125.6 (C-2',6'), 124.0, 114.1 (C-2,6), 43.7 (CH₂), 34.5 (C), 31.3 (3CH₃). HPLC gradient of 15-95% CH₃CN/H₂O in 5 min, tr= 4.67 min, m/z [M+H]⁺= 283. Theoretical elemental analysis C₁₈H₂₂N₂O: C, 76.56; H, 7.85; N, 9.92; experimental elemental analysis C, 76.71; H, 8.10; N, 9.94.

### 4) Formula (III): (R) 3-((4-benzyl-2-oxooxazolidin-3-yl)methyl) methyl benzoate

Potassium *tert*-butoxide (0.6 g, 5.24 mmol) is added to a solution of (*R*)-(-)-4-benzyl-3-propionyloxazolidin-2-one (0.12 g, 5.24 mmol) in anhydrous THF (13 ml), and the reaction mixture is stirred for 45 min at room temperature and under N₂ atmosphere. Then, methyl 3-(bromomethyl)benzoate (1 g, 4.36 mmol) is added and it is stirred for 6 h at room temperature. Subsequently, a saturated ammonium chloride solution is added to the reaction mixture and is extracted with AcOEt (20 ml). The organic extracts are dried over anhydrous MgSO₄, and the solvent is removed at reduced pressure. The crude reaction product is purified by column chromatography (7:3 hexane/ethyl acetate), obtaining (R) 3-((4-benzyl-2-oxooxazolidin-3-yl)methyl)methyl benzoate as a white solid (0.432 g, 62%). MP 62°C. ¹H NMR (300 MHz, CDCl₃) δ 8.06 - 7.95 (m, 1 H), 7.90 (s, 1 H), 7.54 - 7.40 (m, 2H), 7.36 - 7.22 (m, 3H), 7.12 - 7.02 (m, 2H), 4.86 (d, *J* = 15.4 Hz, 1 H), 4.18 (m, 2H), 4.10 - 4.02 (m, 1 H), 3.93 (s, 3H, CH₃), 3.89 - 3.77 (m, 1H), 3.09 (dd, *J*= 13.6, 4.7 Hz, 1H, H-6), 2.66 (dd, *J*= 13.6, 8.9 Hz, 1H, H-6). ¹³C NMR (75 MHz, CDCl₃) δ 166.5 (C-20), 157.9 (C-2), 136.4 (C-7), 135.4 (C-14), 132.4 (C-19), 130.8 (C-16), 129.3 (C-9), 129.1 (C-11), 129.0 (C-18), 128.9 (C-8), 127.1 (C-10), 67.0 (C-5), 55.5 (C-4), 51.9 (C-21), 45.9 (C-13), 38.3 (C-6). HPLC-MS gradient of 15-95% CH₃CN/H₂O in 5 min, tr= 4.8 min, m/z [M+H]⁺= 326. Theoretical elemental analysis C₁₉H₁₉NO₄: C, 70.14; H, 5.89; N, 4.31; experimental elemental analysis C, 70.31; H, 5.67; N, 4.60.

### Example 2. Preparing and obtaining (R)-3-((4-benzyl-2-oxooxazolidin-3-yl)methyl)-N-(4-((4-(trifluoromethyl)benzyl)carbamoyl)phenyl)benzamide (2), of formula (V)

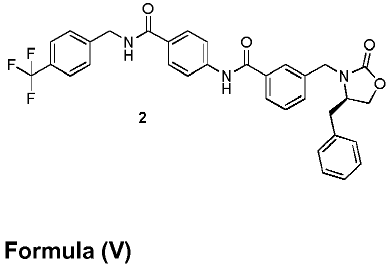

Al(CH₃)₃ in 2.0 M heptane (0.30 ml, 0.61 mmol), under N₂ atmosphere, is added to a solution of (*R*)-3-((4-benzyl-2-oxooxazolidin-3-yl)methyl)methyl benzoate (0.10 g, 0.31 mmol) and 4-amino-N-(4-(trifluoromethyl)benzyl)benzamide (0.18 g, 0.61 mmol); the reaction mixture is heated at 125°C for 35 min in a microwave reactor. The crude reaction product is cooled in an ice-H₂O mixture and acidified with HCl (1N) until effervescence stops. Then, it is extracted with diethylether (30 ml), the organic extracts are dried over anhydrous MgSO₄ and the solvent is removed at reduced pressure. The crude reaction product is purified by chromatography (6:4 Hex/AcOEt), obtaining **2** as a white solid (0.09 g, 50%). ¹H NMR (300 MHz, DMSO-*d*₆) δ 10.49 (s, 1 H, NH), 9.07 (t, *J* = 6.1 Hz, 1 H, CH₂NH), 7.98 - 7.77 (m, 6H), 7.69 - 7.59 (m, 2H), 7.53 - 7.42 (m, 5H), 7.35 - 7.10 (m, 5H), 4.68 (d, *J* = 15.6 Hz, 1 H, H-13) 4.55 (d, *J* = 5.8 Hz, 2H, CH₂NH), 4.41 (d, *J* = 15.6 Hz, 1H, H-13), 4.22 - 4.12 (m, 1H, H-5), 3.92 - 3.81 (m, 1H, H-4), 3.05 (dd, *J* = 13.5, 4.2 Hz, 1 H, H-6), 2.75 (dd, *J* = 13.6, 8.1 Hz, 1 H, H-6). ¹³C NMR (75 MHz, DMSO) δ 165.8 (CO), 165.7 (CO), 157.7 (CO), 144.7, 141.9, 137.3, 136.1, 135.1, 131.0, 129.2 (2C), 129.0, 128.7, 128.5 (3C),128.0 (2C), 127.9 (3C), 127.85, 127.6, 126.8, 126.7, 125.2 (CF₃), 119.5, 66.3 (C-5), 55.3 (C-4), 45.1 (C-13), 42.3 (NHCH₂), 37.2 (C-6). HPLC gradient of 15-95% CH₃CN/H₂O in 10 min, tr= 10.21 min, m/z [M+H]⁺= 589. Theoretical elemental analysis C₃₃H₂₈F₃N₃O₄: C, 67.45; H, 4.80; N, 7.15; experimental elemental analysis C, 67.69; H, 5.00; N, 7.14.

### 1) Intermediate (B): 4-[N-(tert-butoxycarbonyl)amino]-N-(4-trifluoromethyl)benzylamide

4-[(*tert*-butoxycarbonyl)amino] benzoic acid (0.10 g, 0.42 mmol), THF, PyBOP (0.26 g, 0.51 mmol), HOBt (0.07 g, 0.51 mmol), and Et₃N (0.07 ml, 0.51 mmol), 4-(trifluoromethyl)-benzylamine (0.07 ml, 0.51 mmol). 0.14 g, 84% yield. ¹H NMR (300 MHz, DMSO-*d*₆) δ 9.61 (s, 1 H, NH), 8.96 (t, *J* = 6.0 Hz, 1 H, NHCH₂), 7.81 (d, *J* = 8.8 Hz, 2H, CHar), 7.68 (d, *J* = 8.2 Hz, 2H, CHar), 7.56 - 7.47 (m, 4H, CHar), 4.52 (d, *J =* 5.8 Hz, 2H, NHCH₂), 1.47 (s, 9H, CH₃). ¹³C NMR (75 MHz, DMSO) δ 165.8 (CO), 152.5 (CO), 144.7 (Car), 142.4 (Car), 128.0 (Car), 127.8 (2CHar), 127.3 (2CHar), 125.2 (Car), 125.1 (2CHar), 125.1, 125.0, 117.1 (2CHar), 79.4 (CF₃), 42.2 (C(CH₃)₃), 28.0 (CH₃). HPLC gradient of 10-100% CH₃CN/H₂O in 5 min, tr= 5.24 min, m/z [M+H]⁺= 396. Theoretical elemental analysis C₂₀H₂₁F₃N₂O₃: C, 60.91; H,5.37; N, 7.10; experimental elemental analysis C, 61.10; H, 5.25; N, 6.88.

### 2) Formula (II): 4-amino-N-(4-(trifluoromethyl)benzyl)benzamide

4-[*N*-(*tert*-butoxycarbonyl)amino]-*N*-(4-trifluoromethyl)benzylamide (0.20 g, 0.51 mol), DCM (20 ml), trifluoroacetic acid (20 ml). 0.14 g, 92% yield. ¹H NMR (300 MHz, CDCl₃) δ 7.63 (d, *J =* 8.3 Hz, 2H, H-3',5'), 7.58 (d, *J* = 7.8 Hz, 2H, H-2,6), 7.44 (d, *J* = 7.8 Hz, 2H, H-3,5), 6.65 (d, *J* = 8.3 Hz, H-2',6'), 6.42 (s, 1H, NHCH₂), 4.67 (d, *J* = 5.8 Hz, 2H, NHCH₂), 4.34 - 3.49 (bs, 2H, NH). ¹³C NMR (75 MHz, CDCl₃) δ 167.2 (CO), 149.8, 142.8, 128.8 (C-3',5'), 127.9 (C-3,5), 125.7 (C-2,6), 125.6, 125.5, 125.5, 123.4, 114.1 (C-2',6'), 43.4 (CH₂). HPLC gradient of 15-95% CH₃CN/H₂O in 5 min, tr= 4.21 min, m/z [M+H]⁺= 295.

### Example 3. Preparing and obtaining (R)-3-((4-benzyl-2-oxooxazolidin-3-yl)methyl)-N-(4-(benzylcarbamoyl)phenyl)benzamide (3), of formula (VI)

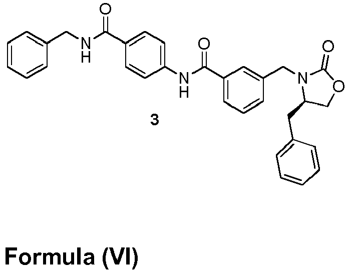

Al(CH₃)₃ in 2.0 M heptane (0.18 ml, 0.36 mmol), under N₂ atmosphere, is added to a solution of (*R*)-3-((4-benzyl-2-oxooxazolidin-3-yl)methyl)methyl benzoate (0.06 g, 0.18 mmol) and the compound 4-amino-*N*-benzylbenzamide (0.081 g, 0.36 mmol) in anhydrous THF (10 ml); the reaction mixture is heated at 125°C for 35 min in a microwave reactor. The crude reaction product is cooled in an ice-H₂O mixture and HCl (1 N) is added dropwise until effervescence stops. Then, it is extracted with diethylether (20 ml), the organic extracts are dried over anhydrous MgSO₄, and the solvent is removed at reduced pressure. The crude reaction product is purified by chromatography (6:4 Hex/AcOEt), obtaining 3 as a white solid (0.04 g, 40%). ¹H NMR (300 MHz, DMSO-*d*₆) δ 10.47 (s, 1 H, NH), 8.95 (t, *J* = 6.0 Hz, 1 H, NHCH₂), 8.04 - 7.78 (m, 6H), 7.64 - 7.48 (m, 4H), 7.45 - 7.13 (m, 8H), 4.68 (d, *J* = 15.6 Hz, 1 H), 4.47 (d, *J =* 6.0 Hz, 2H, NHCH₂), 4.41 (d, *J* = 15.9 Hz, 1 H), 4.22 - 4.18 (m, 1 H), 4.08 - 3.99 (m, 1 H), 4.03 - 3.83 (m, 1H, H-4), 3.05 (dd, *J*= 13.7, 4.2 Hz, 1H, H-6), 2.75 (dd, *J*= 13.5, 8.2 Hz, 1H, H-6). ¹³C NMR (75 MHz, DMSO-*d*₆) δ 165.6 (2CO), 139.8 (CO), 137.3, 136.1, 135.1, 131.0, 129.2 (2C), 128.7, 128.5 (2C), 128.2 (3C), 127.9 (2C), 127.2 (3C), 126.8, 126.7 (2C), 119.5 (2C), 66.3 (C-5), 55.3 (C-4), 45.1 (C-13), 42.5 (NHCH₂), 37.2 (C-6). Theoretical elemental analysis C₃₂H₂₉N₃O₄: C, 73.97; H, 5.63; N, 8.09; experimental elemental analysis C, 73.88; H, 5.66; N, 8.08.

### 1) Intermediate (B): tert-butyl (4-(benzylcarbamoyl)phenyl)carbamate

4-[(*tert*-butoxycarbonyl)amino] benzoic acid (0.20 g, 0.84 mmol), THF (20 ml), PyBOP (0.53 g, 1.01 mmol), HOBt (0.14 g, 1.01 mmol), Et₃N (0.14 ml, 1.01 mmol), benzylamine (0.11 ml, 1.01 mmol). 0.19 g, 80% yield. ¹H NMR (300 MHz, DMSO-*d*₆) δ 9.60 (s, 1 H, NH), 8.86 (t, *J* = 5.9 Hz, 1 H, NHCH₂), 7.80 (d, *J* = 8.7 Hz, 2H, H-2,6), 7.52 (d, *J* = 8.7 Hz, 2H, H-3,5), 7.38 - 7.18 (m, 5H), 4.45 (d, *J* = 5.9 Hz, 2H, NHCH₂), 1.47 (s, 9H, 3CH₃). ¹³C NMR (75 MHz, DMSO) δ 165.7 (CO), 152.5 (CO), 142.3, 139.8, 128.2 (C-2,6), 128.0 (C-3,5), 127.6, 127.1, 126.1, 117.1, 79.4 (C(CH₃)₃), 42.5 (NHCH₂), 28.0 (3CH₃). Theoretical elemental analysis C₁₉H₂₂N₂O₃: C, 69.92; H, 6.79; N, 8.58; experimental elemental analysis C, 70.16; H, 6.49; N, 8.58.

### 2) Formula (II): 4-amino-N-benzylbenzamide

*Tert*-butyl (4-(benzylcarbamoyl)phenyl)carbamate (0.35 g, 1.07 mmol), DCM (20 ml), trifluoroacetic acid (20 ml). 0.22 g, 92% yield. ¹H NMR (300 MHz, DMSO-*d*₆) δ 8.55 (t, *J* = 5.9 Hz, 1 H, NHCH₂), 7.62 (d, *J* = 8.6 Hz, 2H, H-2,6), 7.40 - 7.17 (m, 5H), 6.54 (d, *J =* 8.6 Hz, 2H, H-3,5), 5.60 (s, 2H, NH₂), 4.42 (d, *J =* 5.9 Hz, 2H, NHCH₂). ¹³C NMR (75 MHz, DMSO-*d*₆) δ 166.2 (CO), 151.6, 140.3, 128.7 (C-2,6), 128.1, 127.1, 126.5, 121.0, 112.5 (C-3,5), 42.3 (CH₂). Theoretical elemental analysis C₁₄H₁₄N₂O: C, 74.31; H, 6.24; N, 12.38; experimental elemental analysis C, 74.39; H, 6.43; N, 12.55.

### Example 4. Preparing and obtaining (R)-3-((4-benzyl-2-oxooxazolidin-3-yl)methyl)-N-(4-((cyclohexylmethyl)carbamoyl)phenyl)benzamide. (4), of formula (VII)

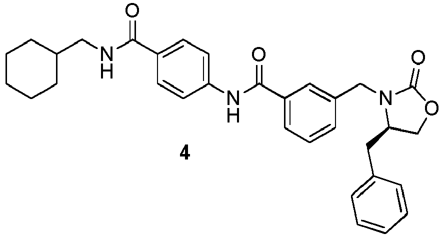

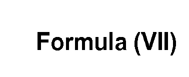

Al(CH₃)₃ in 2.0 M heptane (0.18 ml, 0.36 mmol), under N₂ atmosphere, is added to a solution of (*R*)-3-((4-benzyl-2-oxooxazolidin-3-yl)methyl)methyl benzoate (0.06 g, 0.18 mmol) and the compound 4-amino-*N*-(cyclohexylmethyl)benzamide (0.12 g, 0.36 mmol); the reaction mixture is heated at 125°C for 35 min in a microwave reactor. The crude reaction product is cooled in an ice-H₂O mixture and acidified with HCl (1 N) until effervescence stops. Then, it is extracted with diethylether (2 x 20 ml), the organic extracts are dried over anhydrous MgSO₄ and the solvent is removed at reduced pressure. The crude reaction product is purified by chromatography (6:4 Hex/AcOEt), obtaining compound **4** as a white solid (0.04 g, 42%). ¹H NMR (300 MHz, CDCl₃) δ 9.08 (s, 1H, NH), 7.85 - 7.74 (m, 1H), 7.78 - 7.61 (m, 5H), 7.45 - 7.16 (m, 5H), 7.09 - 6.96 (m, 2H), 6.50 (t, *J* = 6.0 Hz, 1 H, CH₂NH), 4.73 - 4.62 (m, 1 H), 4.21 - 4.06 (m, 2H), 4.00 (m, 1 H), 3.93 - 3.79 (m, 1H, H-4), 3.25 (t, *J* = 6.0 Hz, 2H, CH₂NH), 3.03 (dd, *J*= 13.6, 4.9 Hz, 1H, H-6), 2.63 (dd, *J* = 13.6, 8.6 Hz, 1H, H-6), 1.82 - 1.52 (m, 6H), 1.32 - 1.10 (m, 4H), 0.93 (m, 2H). ¹³C NMR (75 MHz, CDCl₃) δ 166.9 (CO), 165.5 (CO), 158.5, 140.8, 136.8, 135.4, 135.3, 131.4, 130.5, 129.3, 129.0, 127.9 (4C), 127.3 (2C), 127.0, 126.6, 119.8, 119.7 (2C), 67.2 (C-5), 55.7 (C-4), 46.3 (CH₂NH), 46.1 (C-13), 38.8 (C-6), 38.0 (CH), 30.9 (2CH₂), 26.4 (CH₂), 25.8 (2CH₂). HPLC-MS gradient of 40-95% CH₃CN/H₂O in 10 min, tr= 5.71 min, m/z [M+H]⁺= 526. Theoretical elemental analysis C₃₂H₃₅N₃O₄: C, 73.12; H, 6.71; N, 7.99; experimental elemental analysis C, 73.44; H, 6.58; N, 7.52.

### 1) Intermediate (B): tert-butyl (4-((cyclohexylmethyl)carbamoyl)phenyl)carbamate

4-[(*tert*-butoxycarbonyl)amino] benzoic acid (0.14 g, 0.57 mmol), DMF (20 ml), EDCI (0.17 g, 0.88 mmol), HOBt (0.12 g, 0.88 mmol), and Et₃N (0.12 ml, 0.88 mmol), hexahydrobenzylamine (0.11 ml, 0.88 mmol), and the reaction mixture is stirred for 12 h at room temperature. The solvent is removed at reduced pressure, the crude reaction product is redissolved in DCM (20 ml) and acidified with 1 N HCl (10 ml). Then, it is extracted with DCM (2 x 20 ml), the organic extracts are dried over anhydrous MgSO₄, and the solvent is removed at reduced pressure. Finally, it is purified by biotage obtaining **II** as a white solid (0.06 g, 31%). ¹H NMR (300 MHz, DMSO-*d*₆) δ 9.56 (s, 1 H, NH), 8.24 (t, *J* = 5.8 Hz, 1 H, NHCH₂), 7.74 (d, *J* = 8.7 Hz, 2H, H-2,6), 7.49 (d, *J* = 8.6 Hz, 2H, H-3,5), 3.05 (c, *J* = 6.2 Hz, 2H, NHCH₂), 1.67 (m, 5H), 1.47 (s, 9H), 1.24 - 1.01 (m, 4H), 0.90 (m, 2H). ¹³C NMR (75 MHz, DMSO-*d*₆) δ 165.7 (CO), 152.6 (CO), 142.0, 128.06, 127.9 (C-2,6), 117.0 (C-3,5), 79.4 (C(CH₃)₃), 45.3 (NHCH₂), 37.5 (C-1'), 30.5 (C-3',5')), 28.0 (3CH₃), 26.1 (C-4'), 25.4 (C-2',6').

### 2) Formula (II): 4-amino-N-(cyclohexylmethyl)benzamide

*Tert*-butyl (4-((cyclohexylmethyl)carbamoyl)phenyl)carbamate (0.20 g, 0.60 mmol), DCM (20 ml), trifluoroacetic acid (20 ml). 0.11 g colorless oil, 80% yield. ¹H NMR (300 MHz, DMSO-d₆) δ 7.92 (t, *J* = 5.8 Hz, 1 H, CH₂NH), 7.54 (d, *J* = 8.6 Hz, Ar 2,6-H), 6.50 (d, *J* = 8.6 Hz, Ar 3,5-H), 5.60 - 5.48 (m, 2H, NH₂), 3.08 - 2.92 (m, 2H, CH₂NH), 1.66 (m, 5H), 1.47 (m, 1H), 1.13 (m, 3H), 0.95 - 0.77 (m, 2H). HPLC-MS gradient of 15-95% CH₃CN/H₂O in 10 min, tr= 7.35 min, m/z [M+H]⁺= 233.

### Example 5- Preparing and obtaining (R)-3-((4-benzyl-2-oxooxazolidin-3-yl)methyl)-N-(4-(pentylcarbamoyl)phenyl)benzamide (5), of formula (VIII)

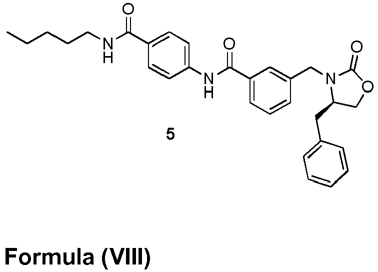

Al(CH₃)₃ in 2.0 M heptane (0.30 ml, 0.61 mmol), under N₂ atmosphere, is added to a solution of (*R*)-3-((4-benzyl-2-oxooxazolidin-3-yl)methyl)methyl benzoate (0.10 g, 0.31 mmol) and the compound 4-amino-*N*-pentylbenzamide (0.13 g, 0.61 mmol); the reaction mixture is heated at 125°C for 35 min in a microwave reactor. The crude reaction product is cooled in an ice-H₂O mixture and acidified with HCl (1N), until effervescence stops. Then, it is extracted with diethylether (2 x 20 ml), the organic extracts are dried over anhydrous MgSO₄, and the solvent is removed at reduced pressure. The crude reaction product is purified by chromatography (6:4 Hex/AcOEt), obtaining **5** as a white solid (0.07 g, 45%). ¹H NMR (300 MHz, DMSO-d₆) δ 10.46 (s, 1 H, NH), 8.35 (t, *J* = 5.6 Hz, 1 H, CH₂NH), 7.91 (m, 1 H), 7.86 (s, 5H), 7.58 - 7.51 (m, 2H), 7.32 - 7.17 (m, 5H), 4.69 (d, *J =* 15.7 Hz, 1 H), 4.42 (d, *J =* 15.6 Hz, 1 H), 4.23 (m, 1 H), 4.06 (m, 1 H), 4.00 - 3.89 (m, 1 H, H-4), 3.25 (c, *J* = 6.7 Hz, 2H, NHCH₂), 3.06 (dd, *J*_{ab}= 13.6, *J*ₐ₄= 4.2 Hz, 1H, H-6), 2.77 (dd, *J*_{ba}= 13.6, *J*_{b4}=8.1 Hz, 1H, H-6), 1.52 (m, 2H), 1.42 - 1.18 (m, 4H), 0.95 - 0.79 (m, *J*= 6.8 Hz, 3H (32)). ¹³C NMR (75 MHz, DMSO-*d*₆) δ 166.0 (CO), 165.9 (CO), 158.1, 141.9, 137.6, 136.5, 135.5, 131.4 130.1, 129.6 (2C), 129.1, 128.9 (2C), 128.2 (2C), 127.6, 127.2, 127.1, 119.8 (2C), 66.7 (C-5), 55.7 (C-4), 45.5 (C-13), 40.7 (CH₂), 37.6 (C-6), 29.2 (CH₂), 29.1 (CH₂), 22.3 (CH₂), 14.3 (CH₃). HPLC-MS gradient of 40-95% CH₃CN/H₂O in 10 min, tr= 5.09 min, m/z [M+H]⁺= 500. Theoretical elemental analysis C₃₀H₃₃N₃O_{4:} C, 72.12; H, 6.66; N, 8.41; experimental elemental analysis C, 72.40; H, 6.83; N, 8.68.

### 1) Intermediate (B): tert-butyl(4-(pentylcarbamoyl)phenylcarbamate

4-[(*tert*-butoxycarbonyl)amino] benzoic acid (0.3 g, 1.26 mmol), THF (20 ml), PyBOP (0.78 g, 1.52 mmol), HOBt (0.21 g, 1.52 mmol) and Et₃N (0.21 ml, 1.52 mmol), 1-pentylamine (0.17 ml, 1.52 mmol). 0.12 g, 33% yield. ¹H NMR (300 MHz, CDCl₃) δ 7.70 (d, *J* = 8.6 Hz, 2H_{Ar}), 7.42 (d, *J* = 8.6 Hz, 2H_{Ar}), 6.70 (s, 1 H, NH), 6.08 (s, 1 H, NH), 1.52 (s, 9H, Me), 1.40 - 1.29 (m, 8H), 0.91 (t, *J* = 6.8 Hz, 3H, CH₃). ¹³C NMR (75 MHz, CDCl₃) δ 166.8 (CO), 152.3 (CO), 141.2, 129.0, 127.9 (C-2,6), 117.7 (C-3,5), 81.0 (C(CH₃)₃), 40.0 (CH₂), 29.4 (CH₂), 29.1 (CH₂), 28.2 (CH₂), 22.4 (CH₃), 13.9 (3CH₃).

### 2) Formula (II): 4-amino-N-pentylbenzamide

*Tert*-butyl(4-(pentylcarbamoyl)phenylcarbamate (0.76 g, 3.68 mmol), DCM/TFA (1:1) (40 ml). 0.44 g, 85% yield. MP 99°C. ¹H NMR (300 MHz, CDCl₃) δ 7.58 (d, *J* = 8.6 Hz, 2H, H-2,6), 6.64 (d, *J* = 8.6 Hz, 2H, H-3,5), 6.01 (s, 1 H, NH), 3.40 (td, *J* = 7.2, 5.7 Hz, 2H, NHCH₂CH₂), 1.57 (c, *J* = 7.1 Hz, 2H, NHCH₂CH₂), 1.43 - 1.24 (m, 4H), 0.99 - 0.79 (m, 3H, CH₃). ¹³C NMR (75 MHz, CDCl₃) δ 167.2 (CO), 149.4, 128.5 (C-2,6), 124.4, 114.1 (C-3,5), 40.0 (CH₂), 29.5 (CH₂), 29.1 (CH₂), 22.4 (CH₂), 14.0 (CH₃).

### Example 6. Preparing and obtaining (R)-3-((4-benzyl-2-oxooxazolidin-3-yl)methyl)-N-(4-(dodecylcarbamoyl)phenyl)benzamide (6), of formula (IX)

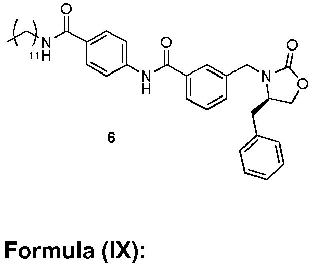

Al(CH₃)₃ in 2.0 M heptane (0.2 ml, 0.36 mmol), under N₂ atmosphere, is added to a solution of (*R*)-3-((4benzyl-2-oxooxazolidin-3-yl)methyl)methyl benzoate (0.06 g, 0.18 mmol) and the compound 4-amino-*N*-dodecylbenzamide (0.11 g, 0.36 mmol); the reaction mixture is heated at 125°C for 35 min in a microwave reactor. The crude reaction product is cooled in an ice-H₂O mixture and acidified with HCl (1 N). Then, it is extracted with diethylether, the organic extracts are dried over MgSO₄ and the solvent is removed at reduced pressure. The crude reaction product is purified by chromatography (6:4 Hex/AcOEt), obtaining compound **6** as a white solid

(0.05 g, 45%). ¹H NMR (300 MHz, CDCl₃) δ 8.69 - 8.56 (m, 1 H), 7.94 - 7.64 (m, 5H), 7.53 - 7.19 (m, 5H), 7.13 - 7.00 (m, 2H), 6.34 - 6.14 (m, 1 H), 4.81 (m, 1 H, CH₂NH), 4.30 - 4.13 (m, 1H), 4.02 (m, 1H), 3.94 - 3.79 (m, 1H), 3.45 (m, 1H, H-4), 3.06 (dd, *J* = 13.6, 4.8 Hz, 1 H, H-6), 2.66 (dd, *J* = 13.6, 8.6 Hz, 1 H, H-6), 1.60 (m, 2H), 1.46 - 1.15 (m, 18H), 0.87 (t, *J* = 6.7 Hz, 3H, CH₃). ¹³C NMR (75 MHz, CDCl₃) δ 166.9 (CO), 165.6 (CO), 158.6, 140.7, 136.7, 135.4, 135.3, 131.4, 130.5, 129.3, 129.0 (3C), 127.8 (2C), 127.3, 127.0, 126.6, 119.9 (2C), 116.7, 67.2 (C-5), 55.7 (C-4), 53.1 (CH₂), 46.1 (C-13), 40.2 (CH₂), 38.7 (C-6), 31.9 (CH₂), 29.6 (4CH₂), 29.3 (3CH₂), 27.0 (CH₂), 22.7 (CH₂), 14.1 (CH₃).

### 1) Intermediate (B): tert-butyl (4-(dodecylcarbamoyl)phenyl)carbamate

4-[(*tert*-butoxycarbonyl)amino] benzoic acid (0.20 g, 0.84 mmol), THF (20 ml), 1-dodecylamine (0.23 ml, 1.01 mmol), EDCI (0.19 g, 1.01 mmol), HOBt (0.14 g, 1.01 mmol), and Et₃N (0.14 ml, 1.01 mmol). 0.22 g, 65% yield. ¹H NMR (300 MHz, CDCl₃) δ 7.69 (d, *J* = 8.6 Hz, 2H, H-2,6), 7.42 (d, *J* = 8.6 Hz, 2H, H-3,5), 6.70 (s, 1 H, NHCO-O), 6.12 (bs, 1 H, NHCO), 3.42 (m, 2H, CH₂NH), 1.61 (m, 2H, CH₂), 1.52 (s, 9H, 3CH₃), 1.42 - 1.17 (m, 18H, 9CH₂), 0.86 - 0.84 (m, 3H, CH₃). ¹³C NMR (75 MHz, CDCl₃) δ 166.8 (CO), 152.3 (CO), 141.3, 128.9, 127.9 (C-2,6), 117.7 (C-3,5), 81.0 (C(CH₃)₃), 40.1 (CH₂N), 31.9 (CH₂), 29.7 (CH₂), 29.6 (CH₂), 29.54 (CH₂), 29.50 (CH₂), 29.3 (3CH₂), 28.3 (3CH₃), 27.0 (CH₂), 22.6 (CH₂), 14.1 (CH₃). Theoretical elemental analysis C₂₄H₄₀N₂O₃: C, 71.25; H, 9.97; N, 6.92; experimental elemental analysis C, 71.08; H, 10.12; N, 6.63.

### 2) Formula (II): 4-amino-N-dodecylbenzamide

*Tert*-butyl (4-(dodecylcarbamoyl)phenyl)carbamate (0.20 g, 0.49 mmol), DCM (20 ml), trifluoroacetic acid (20 ml). 0.14 g, 92% yield. ¹H NMR (300 MHz, CDCl₃) δ 7.59 (d, *J* = 8.6 Hz, 2H, H-2,6), 6.65 (d, *J* = 8.6 Hz, 2H, H-3,5), 6.05 - 5.91 (m, 1 H, NH), 3.40 (c, *J* = 7.1 Hz, 1 H, NHCH₂), 1.65 - 1.50 (m, 2H, NHCH₂CH₂), 1.45 - 1.16 (m, 18H), 0.87 (m, 3H, CH₃). ¹³C NMR (75 MHz, CDCl₃) δ 167.2, 149.3, 128.9, 128.5, 127.8 (C-2,6), 114.1 (C-3,5), 39.9 (NHCH₂), 31.9 (CH₂), 29.8 (CH₂), 29.6 (4CH₂), 29.3 (3CH₂), 27.0 (CH₂), 22.7 (CH₂), 14.09 (CH₃). Theoretical elemental analysis C₁₉H₃₂N₂O: C, 74.95; H, 10.59; N, 9.20; experimental elemental analysis C, 74.65; H, 10.81; N, 9.48.

### Example 7. Preparing and obtaining (R)-3-((4-benzyl-2-oxooxazolidin-3-yl)methyl)-N-(4-(cyclopropylcarbamoyl)phenyl)benzamide (7), of formula (X)

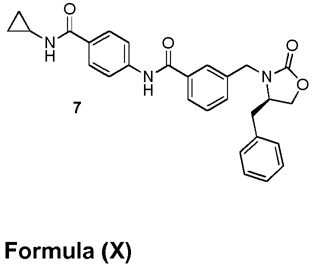

Al(CH₃)₃ in 2.0 M heptane (1.11 ml, 2.24 mmol), under N₂ atmosphere, is added to a solution of (*R*)-3-((4-benzyl-2-oxooxazolidin-3-yl)methyl)methyl benzoate (0. 18 g, 0.56 mmol) and the compound 4-amino-*N*-dodecylbenzamide (0.18 g, 0.67 mmol); the reaction mixture is heated at 125°C for 35 min in a microwave reactor. The crude reaction product is cooled in an ice-H₂O mixture and acidified with HCl (1 N). Then, it is extracted with diethylether, the organic extracts are dried over MgSO₄ and the solvent is removed at reduced pressure. The crude reaction product is purified by chromatography (6:4 Hex/AcOEt), obtaining compound **7** as a white solid (0.05 g, 2%). ¹H NMR (MeOD, 300 MHz) δ 7.90 - 7.88 (m, 1 H), 7.84 (bs, 2H), 7.79 - 7.77 (m, 2H), 7.55 - 7.51 (m, 4H), 7.30 - 7.26 (m, 2H), 7-18 - 7.16 (m, 2H), 4.81 (d, *J=* 4.8 Hz, 1 H), 4.36 (d, *J=* 4.4 Hz 1 H), 4.30 - 4.26 (m, 1 H), 4.15 - 4.12 (m, 1 H), 4.06 - 4.02 (m, 1 H), 3.11 (dd, J= 13.7, 5.1 Hz, 1 H), 2.87 - 2.80 (m, 2H), 0.83 - 0.79 (m, 2H), 0.66 - 0.63 (m, 2H). ¹³C NMR (MeOD, 75 MHz) δ 169.7 (C), 167.2 (C), 159.5 (C), 141.7 (C), 137 (C), 135.9 (C), 135.3 (C), 131.1 (2CH), 129.5 (C), 128.9 (2CH), 128.8 (2CH), 128.4 (2CH), 126.9 (2CH), 126.7 (CH), 119.8 (2CH), 67.2 (CH₂), 55.9 (CH), 45.5 (CH₂), 37.8 (CH₂), 22.6 (CH), 5.14 (2 CH₂).

### 1) Intermediate (B): tert-butyl-4-(cyclopropylcarbamoyl)carbamate

4-[(*tert*-butoxycarbonyl)amino] benzoic acid (0.50 g, 2.11 mmol), DMF (25 ml), cyclopropylamine (0.22 ml, 3.17 mmol), EDCI (0.61 g, 3.17 mmol), HOBt (0.43 g, 3.17 mmol), and Et₃N (0.88 ml, 6.33 mmol). 0.28 g, 48% yield. MP 184°C. ¹H NMR (MeOD, 300 MHz) δ 7.72 (d, *J* = 8.8 Hz, 2 H), 7.48 (d, *J* = 8.8 Hz, 2H), 2.82 (qd, *J* = 7.4, 3.9 Hz, 1 H), 1.52 (s, 9H), 0.93 - 0.73 (m, 2H), 0.69 - 0.50 (m, 2H). ¹³C NMR (MeOD, 75 MHz) δ 164.6 (NHCOAr), 155.2 (NHCOO), 144.5 (C), 139.15 (C), 129.6 (2CH), 119.1 (2CH), 81.6 (C), 29.0 (3CH₃), 24.3 (CH), 6.95 (2CH₂). HPLC-MS gradient of 15-95% CH₃CN/H₂O in 10 min, tr= 7.84 min, m/z [M+H]⁺= 277. Theoretical elemental analysis C₁₅H₂₀N₂O₃: C, 65.2; H, 7.3; N, 10.14; experimental elemental analysis C, 65.18; H, 6.94; N, 10.08.

### 2) Formula (II): 4-amino-N-cyclopropyl benzamide

*Tert*-butyl (4-(cyclopropylcarbamoyl)carbamate (0.55 g, 1.99 mmol), DCM (30 ml), trifluoroacetic acid (30 ml). 0.22 g, 63% yield. MP 142°C ¹H NMR (300 MHz, MeOD) δ 7.57 (d, *J=* 8.7 Hz, 2H), 6.64 (d, *J=* 8.7 Hz, 2H), 4.86 (s, 4H), 2.85 - 2.71 (m, 1 H), 0.83 - 0.70 (m, 2H), 0.65 - 0.53 (m, 2H). ¹³C NMR (75 MHz, MeOD) δ 172.1 (NHCOAr), 153.2 (C), 129.9 (C), 123.1 (2CH), 114.6 (2CH), 23.8 (CH), 6.6 (2CH₂). HPLC-MS gradient of 15-95% CH₃CN/H₂O in 10 min, tr= 1.2 min, m/z [M+H]⁺= 177. Theoretical elemental analysis C₁₀H₁₂N₂O: C, 68.16; H, 6.86; N, 15.90; experimental elemental analysis C, 67.97; H, 6.80; N, 15.75.

### B. IN VITRO ASSAY ON ACTIVATION OF PPAR ALPHA AND GAMMA RECEPTORS

### Cell transfection and luciferase reporter gene assay

### Preparation and reagents

The reporter gene assay is an *in vitro* method used to determine and quantify the existence of physical interactions between proteins, useful for confirming in this case the interaction of the transcription factors PPAR-alpha with co-activator SRC-1, and PPAR-gamma with PPRE in MCF-7 cells.

Commercial compound GW7647 was as the positive control for PPAR-alpha and Rosiglitazone was used as the positive control for PPAR-gamma Tocris Bioscience (Cookson Lted. Bristol, UK). For the *in vitro* experiments, with cell culture all the compounds were dissolved and diluted in dimethylsulfoxide (DMSO) (Sigma Aldrich Spain).

DNA constructs: Eukaryotic pSG5 expression vector (4100 base pairs, Stratagene Co.) was used as the cDNA expression vehicle for human PPAR-alpha receptor and PPAR-gamma receptor. This construction was used for the *in vivo* overexpression of the protein in mammalian cells.

Four copies of the human CPTI gene DR1-type RE (sequence GTAGGGAAAAGGTCA) (Ref: Tinahones FJ, Moreno-Santos I, Vendrell J, Chacon MR, Garrido-Sanchez L, Garcia-Wasntes E, and Macias-Gonzalez M. Obesity 2012, 20: 488-497) were individually fused with the thymidine kinase promoter in the pGL-2 Basic vector (5598 base pairs, Promega Co.), which lacks a eukaryotic promoter and contains a firefly (*Photinus pyralis*) luciferase reporter gene. This vector is widely used in the quantitative analysis of factors capable of regulating gene expression in mammalian cells. This pGL-2 Basic vector also has an ampicillin resistance region (gene for β-lactamase).

Human MCF-7 breast cancer cells were cultured in 6-well plates (10⁵ cells/ml) and grown overnight for stabilization in Dulbecco's modified Eagle medium (DMEM) free of red phenol supplemented with 5% charcoal stripped fetal bovine serum or FBS.

### Cell transfection

### Liposomes containing DNA plasmids were formed by incubating:

- 1 µg of an expression vector for PPAR-alpha, RXR-alpha and wild-type SRC-1, or 1 µg of expression vector for PPAR-gamma and PPRE wild-type;
- 1 µg of luciferase reporter plasmid,
with 10 µg of *N*-[1-(2,3-dioleoyloxy)propyl]-*N*,*N*,*N*-trimethylammonium methylsulfate (DOTAP, Roche) for 15 minutes at room temperature in a total volume of 100 µl. After diluting with 900 µl of red phenol-free DMEM, the liposomes were added to the cells. The red phenol-free DMEM supplemented with 500 µl of 15% charcoal stripped FBS was added 4 h after transfection. At this point, the cells were treated 16 hours with different concentrations in DMSO of the different compounds on the evaluation as indicated.

The cells were lysed 16 hours after the start of stimulation with a Roche Diagnostics GmbH reporter gene lysis buffer (50 ml of buffer 5x), following the company's protocol.

The chemiluminescence assay which allows a quantitative determination of luciferase activity in transfected cells was carried out by also following the supplier's indications (Luciferase Reporter Gene Assay, constant light signal of Roche Diagnostics GmbH). The presence of this enzyme is detectable in transfected cell extracts as a result of the bioluminescence thereof: the reaction catalyzed by luciferase converts luciferin into oxyluciferin in the presence of ATP, Mg 2+ and O₂, and furthermore produces visible light photons.

Luciferase activity was normalized with respect to protein concentration (previously obtained following the Bradford method with the reagent kit, Quick Start Bradford Protein Assay Kit 1x commercially supplied by Bio-Rad Laboratories Inc. and using a VERSAmax® microplate reader of Molecular Devices Corp.), and the induction factors were calculated as the ratio of luciferase activity of the ligand-stimulated cells with respect to the solvents (González MM and Carlberg C., JBC, 2002, 277: 18501-9), as shown in Table 1 corresponding to the PPAR-alpha and PPAR-gamma affinity results.

### C. IN VIVO ASSAY ON FOOD INGESTION

### Studies on food ingestion: acute treatment

Male Wistar albino rats (Rattus Norvegicus) from Charles Rivers Laboratories España, S.A. (Barcelona, Spain), were used, having weights ranging between 300-350 g at the start of the experiments. The animals were caged individually, in controlled temperature (22°C) and moisture (55%) conditions under a 12-hour light/dark cycle (light off from 8:00 AM to 8:00 PM). The rats had free access to food and water in their corresponding cages. All the training and experimental sessions were carried out after 8:00 PM hours.

All the drugs were prepared in fresh conditions in a solution consisting of 5% Tween-80 in sterile physiological serum. The different doses were prepared in different vials such that Tween-80 was added after weighing the necessary amount of compound. The compound, with its corresponding volume of surfactant, was sonicated and homogenized (with the aid of a magnetic stirrer) for 15-30 minutes. Then, the necessary volume of serum was added until completing the solution.

The compounds were administered intraperitoneally (i.p.) in an acute manner in an administration volume of 1 ml per kg of animal.

Before starting the ingestion experiments, the animals were habituated to being handled. For this purpose, 48-72 hours before each experimental session, the rats were placed in cages where the base material (sawdust) was removed, and small containers with food were placed in the cages for 4 hours. After this initial habituation phase, the animals were deprived of food for 24 hours, always having free access to water.

After said fasting period, the different treatment groups were established, and the base material was removed from the cages, weighing the animals and weighing the small containers with food (commercial laboratory feed). Additionally, the water container was weighed. 15 minutes after administering the compounds, the respective weighed food container was given to each animal, and it was again weighed at different times (30, 60, 120 and 240 minutes). In one case it was weighed 24 hours after being given.

This data was used to calculate food consumption food in those time intervals. Food consumption was represented as relative accumulated consumption (g of food per kg of animal). The water containers were also weighed at the end of the experiment.

### Biological results

The results of the biological assays are shown below.

**Table 1. Activation concentration values 50 (EC50) for different specific PPAR-alpha activating compounds, specific PPAR-gamma activating compounds or pan activators.**

| **Compound** | **PPAR-alpha EC50 (nM)** | **PPAR-gamma EC50 (nM)** |
|---|---|---|
| GW7647 | 65±1 | ------- |
| Rosiglitazone | ------- | 870±11 |
| 1 | 670±157 | 1289+261 |
| 5 | 821±139 | 1622+388 |

Figure 1 shows the results of the acute ingestion experiment in relation to compound 1, where it is seen that said compound is capable of reducing ingestion by about 50% at an effective dose of 3.0 mg/kg, showing an effect 120 minutes after the administration thereof and said effect disappearing 240 minutes after the administration thereof, which makes it interesting for acute administration.

Figure 2 shows the results of the acute ingestion experiment in relation to compound 5 which indicate that said compound is active in a dose-dependent manner, reducing ingestion by about 50%, at an effective dose of 1.0 mg/kg. This compound shows an effect maintained over time after 240 minutes, and it surprisingly still has an effect 24 hours after administration (Figure 3), which makes it interesting for chronic administration.

## Claims

1. A compound of general formula (I) 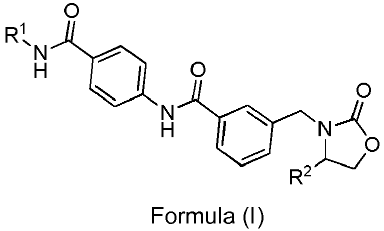 or any of the pharmaceutically acceptable salts, esters, tautomers, solvates and hydrates thereof, where:
R¹ is selected from (C₁-C₁₂) alkyl, cycloalkyl or -(CH₂)-R³ where R³ is cycloalkyl or aryl;
R² is selected from (C₁-C₁₂) alkyl, aralkyl, aryl and amino.

2. The compound according to claim 1, **characterized in that** R² is aralkyl.

3. The compound according to claim 2, **characterized in that** R² is benzyl.

4. The compound according to any one of claims 1-3, **characterized in that** R¹ is cycloalkyl.

5. The compound according to claim 4, **characterized in that** R¹ is selected from cyclopropyl, cyclobutyl, cyclopentyl or cyclohexyl.

6. The compound according to claim 5, **characterized in that** R¹ is cyclopropyl.

7. The compound according to any one of claims 1-3, **characterized in that** R¹ is (C₁-C₁₂) alkyl.

8. The compound according to claim 7, **characterized in that** R¹ is pentyl or dodecyl

9. The compound according to any one of claims 1-3, **characterized in that** R¹ is -(CH₂)-R³, and where R³ is cycloalkyl or aryl.

10. The compound according to claim 9, **characterized in that** R³ is cyclohexyl or aryl in the form of 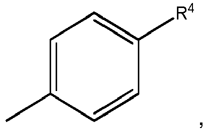 and **characterized in that** R⁴ is hydrogen or a linear or branched alkyl optionally substituted with one or several halogens.

11. The compound according to claim 1, **characterized in that** it is selected from the list consisting of:
a) (*R*)-3-((4-benzyl-2-oxooxazolidin-3-yl)methyl)-*N*-(4-((4-(*tert*-butyl)benzyl)carbamoyl)phenyl) benzamide (**1**), of formula (IV): 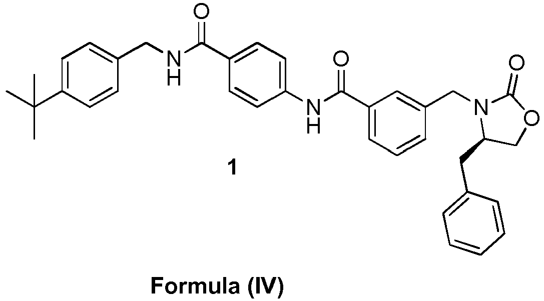
b) (*R*)-3-((4-benzyl-2-oxooxazolidin-3-yl)methyl)-*N*-(4-((4-(trifluoromethyl)benzyl)carbamoyl) phenyl)benzamide (**2**), of formula (V): 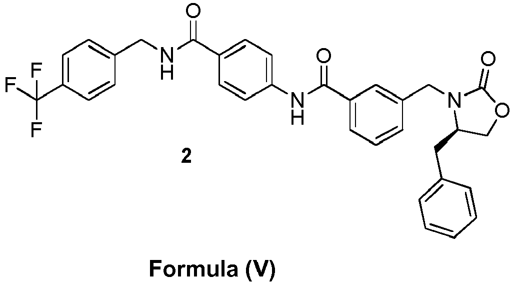
c) (*R*)-3-((4-benzyl-2-oxooxazolidin-3-yl)methyl)-*N*-(4-(benzylcarbamoyl)phenyl)benzamide (**3**), of Formula (VI): 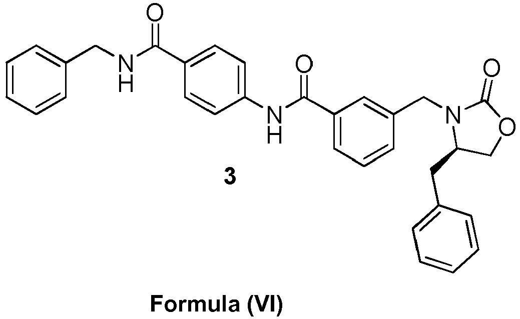
d) (*R*)-3-((4-benzyl-2-oxooxazolidin-3-yl)methyl)-*N*-(4-((cyclohexylmethyl)carbamoyl)phenyl) benzamide (**4**), of Formula (VII): 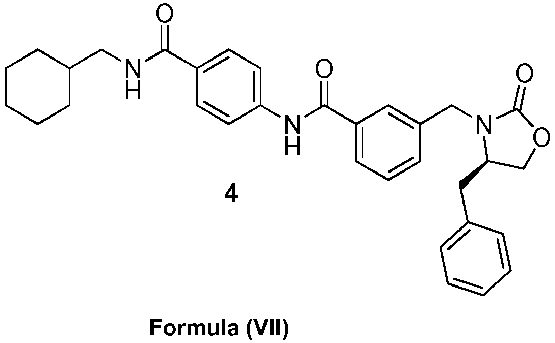
e) (R)-3-((4-benzyl-2-oxooxazolidin-3-yl)methyl)-*N*-(4-(pentylcarbamoyl)phenyl)benzamide (**5**), of formula (VIII): 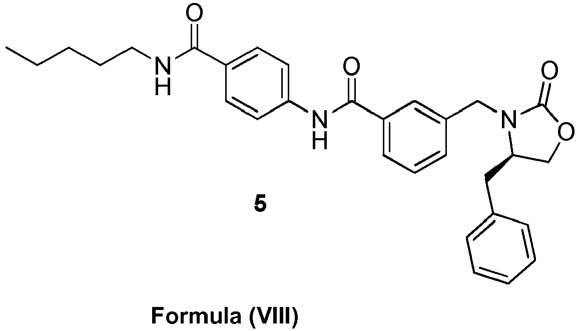
f) (*R*)-3-((4-benzyl-2-oxooxazolidin-3-yl)methyl)-*N*-(4-(dodecylcarbamoyl)phenyl)benzamide (**6**), of formula (IX). 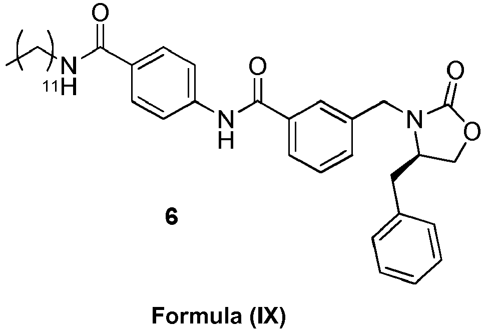
g) (*R*)-3-((4-benzyl-2-oxooxazolidin-3-yl)methyl)-*N*-(4-(cyclopropylcarbamoyl)phenyl)benzamide (**7**), of formula (X): 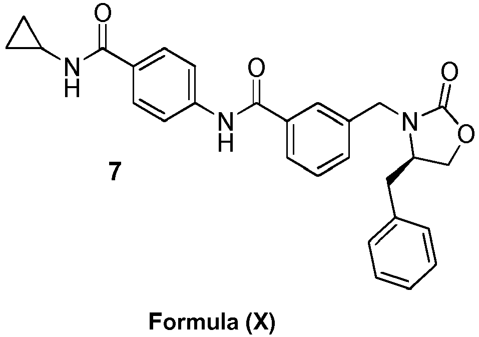 or any of the pharmaceutically acceptable salts, esters, tautomers, prodrugs, solvates and hydrates thereof.

12. The compound according to any of claims 1-11, **characterized in that** is a dual PPAR α and PPAR γ receptor agonist.

13. A pharmaceutical composition comprising a compound according to any of claims 1-12.

14. The pharmaceutical composition according to the preceding claim, **characterized in that** it further comprises a pharmaceutically acceptable vehicle.

15. The pharmaceutical composition according to any of claims 13-14, **characterized in that** it further comprises another active ingredient.

16. A dosage form comprising a compound according to any of claims 1-12, or a pharmaceutical composition according to any of claims 13-15.

17. In *vitro* use of a compound according to any of claims 1-12, a pharmaceutical composition according to any of claims 13-15, or the dosage form according to claim 16, as receptor activators of the α (alpha) and γ (gamma) subtypes of the PPAR receptors.

18. Use of a compound according to any of claims 1-12, a pharmaceutical composition according to any of claims 13-15, or the dosage form according to claim 16, in the preparation of a medicinal product.

19. Use of a compound according to any of claims 1-12, a pharmaceutical composition according to any of claims 13-15, or the dosage form according to claim 16, in the preparation of a medicinal product for the prevention or treatment of a pathology mediated by the α and γ subtypes of the PPAR receptors (peroxisome proliferator-activated receptors).

20. Use of a compound according to any of claims 1-12, a pharmaceutical composition according to any of claims 13-15, or the dosage form according to claim 16, in the preparation of a medicinal product for inducing satiety and controlling ingestion, modulating body fat and regulating lipid and carbohydrate metabolism.

21. Use of a compound according to any of claims 1-12, a pharmaceutical composition according to any of claims 13-15, or the dosage form according to claim 16, in the preparation of a medicinal product for the prevention or treatment of a metabolic disease.

22. Use of a compound, a pharmaceutical composition, or the dosage form according to claim 21, where the metabolic disease is selected from the group consisting of obesity, dyslipidemia, insulin resistance, hyperglycemia and type 2 diabetes.

23. Use of a compound according to any of claims 1-12, a pharmaceutical composition according to any of claims 13-15, or the dosage form according to claim 16, in the preparation of a medicinal product for the prevention or treatment of a cardiovascular disease.

24. Use of a compound, a pharmaceutical composition, or the dosage form according to claim 23, **characterized in that** the cardiovascular disease is selected from atherosclerosis and hypertension.

25. A cosmetic composition comprising a compound according to any of claims 1-12, and at least one cosmetically acceptable excipient and/or adjuvant.

26. A dosage form comprising the cosmetic composition according to claim 25.

27. Use of the cosmetic composition according to claim 25, or a dosage form according to claim 26, for reducing subcutaneous fat.

28. Use of the compound according to any of claims 1-12, of a pharmaceutical composition according to any of claims 13-15, of the dosage form according to claim 16 or of the dosage form according to claim 26 in the preparation of a medicinal product for reducing subcutaneous fat.

29. A nutraceutical composition comprising a compound according to any of claims 1-12.

30. Use of the nutraceutical composition according to claim 29 for reducing subcutaneous fat.

31. A method for obtaining a compound of general formula (I), **characterized in that** it comprises reacting a compound of formula (II) with a compound of formula (III) in the presence of trimethyl aluminum and THF, 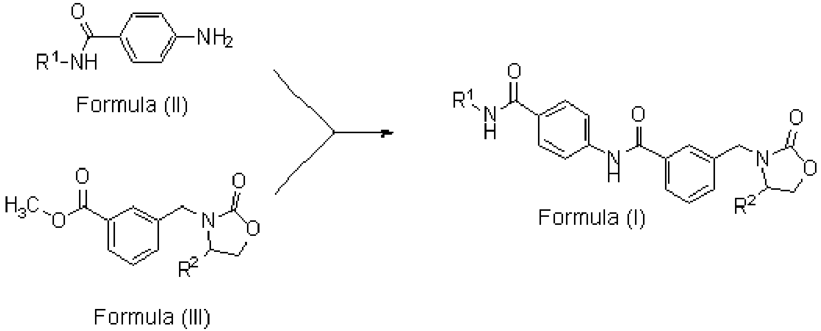 where the substituents are defined as in claim 1.

32. The method for obtaining a compound of general formula (I) according to claim 31, **characterized by** the final reaction of an arylamine with an ester, including the following steps:
a) Protecting the amine of p-amino benzoic acid with Boc anhydride;
b) Reacting protected p-amino benzoic acid with an amine by means of HOBt, EDCI or PyBOP acid activating agent in the presence of triethylamine;
c) Deprotecting the amino group in a CH₂Cl₂/TFA biphasic system to obtain an arylamine;
d) Reacting by means of a nucleophilic substitution of an oxazolidinone enolate on the brominated derivative in the presence of potassium *tert*-butoxide as a base in THF to obtain a methyl ester;
e) Reacting to form the amide bond from methyl ester and arylamine in the presence of trimethyl aluminum at 125°C for 35 min in a microwave reactor. The crude reaction product is cooled in an ice-H₂O mixture and acidified with HCl (1 N), until effervescence stops;
f) Extracting with diethylether (2 x 20 ml), drying over anhydrous MgSO₄ and removing the solvent at reduced pressure;
g) Purifying the crude reaction product by chromatography (6:4 Hex/AcOEt).

33. The method for obtaining a compound of general formula (I) according to claim 32, **characterized in that** the amine is chosen from 4-(*tert*-butyl)-benzylamine, 4-(trifluoromethyl)-benzylamine, benzylamine, hexahydrobenzylamine, 1-pentylamine, 1-dodecylamine, cyclopropylamine, and the ester is methyl 3-(bromomethyl)benzoate.
